(19) Europäisches Patentamt
European Patent Office
Office européen des brevets

(11) Veröffentlichungsnummer: **0 133 674**
**B1**

(12) # EUROPÄISCHE PATENTSCHRIFT

(45) Veröffentlichungstag der Patentschrift: **20.09.89**

(51) Int. Cl.[4]: **A 61 K 49/00**, C 07 D207/46, C 07 D211/94, C 07 G 17/00, C 07 D405/12, C 07 K 15/12// C07D207/16, C07D207/22

(21) Anmeldenummer: **84108900.6**

(22) Anmeldetag: **27.07.84**

(54) **Neue Nitroxylverbindungen, Verfahren zu deren Herstellung und diese enthaltende diagnostische Mittel.**

(30) Priorität: **03.08.83 DE 3328365**

(43) Veröffentlichungstag der Anmeldung: **06.03.85 Patentblatt 85/10**

(45) Bekanntmachung des Hinweises auf die Patenterteilung: **20.09.89 Patentblatt 89/38**

(84) Benannte Vertragsstaaten: **AT BE CH DE FR GB IT LI LU NL SE**

(56) Entgegenhaltungen:
**FR-A- 1 501 115**
**FR-A- 1 570 560**
**FR-A- 2 235 103**
**CHEMICAL ABSTRACTS, Band 88, Nr. 19, 8. Mai 1978, Seite 371, Nr. 134654s, Columbus, Ohio, US; F.M. POULSEN u.a.: "Structural properties of the combining site of anti streptococcus group A antibodies: spin label-hapten studies" & CARLSBERG RES. COMMUN. 1977, 42(5), 369-78**
**DIE PHARMAZIE, Band 35, Heft 1, Januar 1980, Seiten 10-14, Berlin, DD; P. KERTSCHER u.a.: "Synthese einiger spinmarkierter Glycerophospholipide"**
**SYNTHESIS, Nr. 7, Juli 1975, Seiten 462-463, Stuttgart, DE; B.T. GOLDING u.a.: "A short-cut to spin-labelled amides"**
**BIOCHEMISTRY, Band 14, Nr. 12, 17. Juli 1975, Seiten 2754-2760, Columbus, Ohio, US; L.S. JOHNSTON u.a.: "Initial membrane reaction in the biosynthesis of peptidoglycan. Spin-labeled intermediates as receptors for vancomycin and ristocetin"**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Gries, Heinz, Dr.**
**Helmstedter Strasse 19**
**D-1000 Berlin 31 (DE)**
Erfinder: **Niedballa, Ulrich, Dr.**
**Gosslerstrasse 28a**
**D-1000 Berlin 33 (DE)**
Erfinder: **Weinmann, Hanns-Joachim, Dr.**
**Westhofener Weg 27**
**D-1000 Berlin 38 (DE)**

**EP 0 133 674 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

CHEMICAL ABSTRACTS, Band 90, Nr. 17, 23. April 1979, Seite 494, Nr. 137610b, Columbus, Ohio, US; H.O. HANKOVSZKY u.a.: "Nitroxides. II. 1-Oxyl-2,2,5,5-tetramethylpyrroline-3-carboxylic acid derivatives" & ACTA CHIM. ACAD. SCI. HUNG. 1978, 98(3), 339-48
Tetrahedron, vol. 33, 1977, pp. 2969-2980
N.W. Alcock et al., Pergamon Press.

**Beschreibung**

Die Erfindung betrifft den in den Patentansprüchen gekennzeichneten Gegenstand, das heißt Verbindungen der allgemeinen Formel I

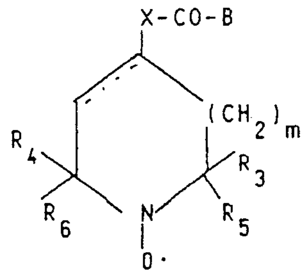

(I)

worin

B einen Protein-, Zucker- oder Lipidrest oder eine Gruppe

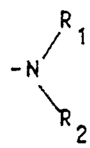

. 

....... eine Einfachbindung oder Doppelbindung bedeutet,

X die Gruppierung $-(CH_2)_n-$ oder falls ....... eine Einfachbindung ist, auch die Gruppierung $-NHCO(CH_2)_n-$ mit n in der Bedeutung von 0 bis 4 darstellt, m die Ziffern 0, 1 oder 2 bedeutet,

$R_1$ einen durch 1 bis 5 Hydroxygruppen, 1 bis 5 $C_2$- bis $C_6$-Acyloxygruppen und/oder 1 bis 3 $C_1$- bis $C_6$-Alkylidendioxygruppen substituierten $C_2$- bis $C_8$-Alkylrest darstellt, $R_2$ die gleiche Bedeutung wie $R_1$ besitzt oder ein Wasserstoffatom oder einen $C_1$- bis $C_4$-Alkylrest bedeutet,

$R_3$ und $R_4$ $C_1$- bis $C_4$-Alkylreste darstellen und

$R_5$ und $R_6$ gegebenenfalls durch 1 bis 3 Hydroxygruppen substituierte $C_1$- bis $C_4$-Alkylreste bedeuten,

zur Verwendung in Diagnostizierverfahren, die am menschlichen oder tierischen Körper vorgenommen werden, diese Verbindungen enthaltende diagnostische Mittel, neue Verbindungen der allgemeinen Formel I sowie Verfahren zur Herstellung dieser neuen Verbindungen.

Die Verbindungen der allgemeinen Formel I tragen als Substituenten B entweder den Rest eines Proteins wie beispielsweise Albumin, Immunglobulin oder monoklonale Antikörper oder den eines Zuckers wie beispielsweise Glucose oder ein Glukosederivat oder den eines Lipids wie beispielsweise Dipalmitoylphosphatidylethanolamin bzw. die Gruppe $-NR_1R_2$. Hierin bedeutet $R_1$ einen durch Hydroxygruppen, Acyloxygruppen oder Alkylidendioxygruppen substituierten Alkylrest mit vorzugsweise 2 bis 8 und insbesondere 2 bis 6 Kohlenstoffatomen. Alle Kohlenstoffatome dieser Alkylreste können Hydroxygruppen tragen, ausgenommen das C-Atom, welches an den Amidstickstoff gebunden ist. Geeignete Substituenten $R_1$ sind beispielsweise: der 2-Hydroxyethylrest, der 2-Hydroxypropylrest, der 2-Hydroxy-1-methyl-ethylrest, der 3-Hydroxypropylrest, der 2,3-Dihydroxypropylrest, der 2-Hydroxy-1-(hydroxymethyl)-ethylrest, der 2-Hydroxybutylrest, der 2,3-Dihydroxybutylrest, der 2,3,4-Trihydroxybutylrest, der 2,3-Dihydroxy-1-hydroxymethylpropylrest, der 2,3,4,5-Tetrahydroxypentylrest oder der 2,3,4,5,6-Pentahydroxyhexylrest. Die Hydroxygruppen können in freier, veresterter oder ketalisierter Form vorliegen. Geeignete Ester sind vorzugsweise solche, die sich von einer Alkancarbonsäure mit 2 bis 6 Kohlenstoffatomen, wie zum Beispiel der Essigsäure, Propionsäure, Buttersäure, Isobuttersäure, Trimethylessigsäure, Valeriansäure oder Capronsäure ableiten. Ketalisierte Hydroxygruppen sind insbesondere die Acetonide.

Der Substituent $R_2$ kann die gleiche Bedeutung wie der Substituent $R_1$ haben. Vorzugsweise ist er aber ein Wasserstoffatom oder eine Alkylgruppe mit bis zu 4 Kohlenstoffatomen, wie die Ethylgruppe, die Propylgruppe, die Isopropylgruppe oder insbesondere die Methylgruppe.

Die Substituenten $R_3$ und $R_4$ stellen niedere Alkylgruppen mit 1 bis 4 Kohlenstoffatomen, wie die Ethylgruppe, die Propylgruppe, die Isopropylgruppe oder insbesondere die Methylgruppe dar.

Die Substituenten $R_5$ und $R_6$ haben vorzugsweise die gleiche Bedeutung wie $R_3$ und $R_4$. Sie können aber auch durch Hydroxygruppen substituierte Alkylreste mit bis zu 4 Kohlenstoffatomen, wie zum Beispiel die Hydroxymethylgruppe, die 1-Hydroxyethylgruppe, die 2-Hydroxyethylgruppe, die 1,2-Dihydroxyethylgruppe, die 1-Hydroxypropylgruppe, die 1,2-Dihydroxypropylgruppe oder die 1,2,3-Trihydroxypropylgruppe darstellen.

m bedeutet vorzugsweise die Ziffer 0 oder 1

In der Gruppierung $(CH_2)_n$ hat n vorzugsweise die Bedeutung von 0 oder 1 ; in der Gruppierung $-NHCO(CH_2)_n-$ hat n vorzugsweise die Bedeutung von 3 oder 4 und insbesondere die Bedeutung von 2.

In der Literatur sind bereits einige unter die allgemeine Formel I fallende Nitroxyle bekannt; allerdings hat ihre angegebene Verwendung nichts mit dem Erfindungsgegenstand, nämlich ihrer Eignung als diagnostische Mittel, zu tun.

So werden in FR-A-2 235 103 und Chemical Abstracts 88, 1978, S. 371, Nr. 134654s 2,2,5,5-Tetramethyl-3-pyrrolin-1-oxyl-3-carbonsäure-saccharide beschrieben, die zur Untersuchung der Struktur und Permeabilität von Membranen, der Morphologie und des Bioabbaus von Sacchariden bzw. als Haptene zur Untersuchung von anti-Streptococcus Gruppe A-Antikörpern verwendet werden.

In Pharmazie 35, 1980, 10 wird die Synthese von Phospholipiden durch Umsetzung eines primären Alkohols eines 2,2,5,5-Tetramethyl-pyrrolin-1-oxyl-Grundkörpers mit einer Phosphorsäure, die für biophysikalische Untersuchungen in biologischen und Modellmembranen verwendet werden sollen, beschrieben.

In Synthesis 1975, 462 und Tetrahedron 33, 1977, 2969 werden die durch Umsetzung von 3,5-Dibrom-4-oxo-2,2,6,6-tetramethylpiperidin-1-oxyl mit Aminen hergestellten und für Untersuchungen der Biochemie von β-Laktam-Antibiotika verwendeten (2-Hydroxyethyl)- und (2,3-Dihydroxypropyl)-amide der 2,2,5,5-Tetramethyl-3-pyrrolin-1-oxyl-3-carbonsäure beschrieben.

In Biochemistry 14, 1975, 2754 wird die Verwendung eines an die Amidgruppe des 2,2,5,5-Tetramethyl-pyrrolin-1-oxyl-3-carbonsäureamids gebundenen Pentapeptids als Rezeptor für Vancomycin und Ristocetin beschrieben.

Es wurde nun gefunden, daß diese Substanzen und die übrigen nicht vorbekannten Verbindungen der allgmeinen Formel I überraschenderweise hervorragend zur Herstellung diagnostischer Mittel geeignet sind, welche bei der NMR-Diagnostik Anwendung finden. Es besteht für vielfältige Zwecke ein Bedarf vor allem an gut verträglichen, aber auch stabilen, gut löslichen und hinreichend selektiven NMR-Diagnostika. Die hier beschriebenen Verbindungen der allgemeinen Formel I — insbesondere diejenigen, in denen die Hydroxygruppen nicht in veresterter oder ketalisierter Form vorliegen — entsprechen diesen Anforderungen.

Beispielsweise eignen sich die an Proteine gebundenen Verbindungen der allgemeinen Formel I insbesondere für die Tumor- und Infarkt-Diagnostik. Für Leber- und Milz-Untersuchungen sind beispielsweise Lipidkonjugate oder Einschlußverbindungen mit Liposomen, die beispielsweise als uni- oder multilamellare Phosphatidylcholin-Cholesterol-Vesikel vorliegen, besonders geeignet.

Die beanspruchten diagnostischen Mittel können enteral oder parenteral, insbesondere intravenös, intraarteriell oder intralumbal, appliziert werden.

Die neuen Verbindungen der allgemeinen Formel I mit B in der Bedeutung einer $-NR_1R_2$-Gruppe können nach einem Verfahren hergestellt werden, dadurch gekennzeichnet, daß man in an sich bekannter Weise

a) eine Verbindung der allgemeinen Formel II

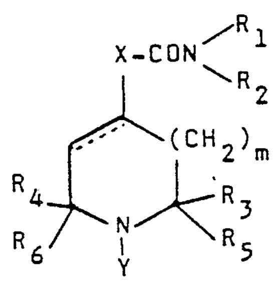

(II)

worin ⋯⋯ , m, X, $R_1$, $R_2$, $R_3$, $R_4$, $R_5$ und $R_6$ die oben genannte Bedeutung besitzen und

Y ein Wasserstoffatom oder eine Hydroxygruppe darstellt, oxidiert, oder

b) eine Carbonsäure der allgemeinen Formel III

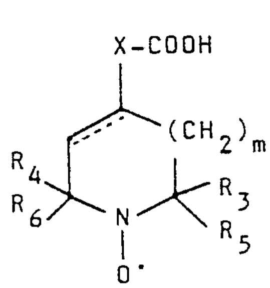

(III)

worin ⋯⋯ , m, X, $R_3$, $R_4$, $R_5$ und $R_6$ die obengenannte Bedeutung besitzen, oder ein reaktionsfähiges Derivat dieser Carbonsäure, mit einem Amin der allgemeinen Formel IV

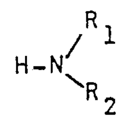

(IV)

worin $R_1$ und $R_2$ die obengenannte Bedeutung besitzen, kondensiert oder

c) eine Carbonsäure der allgemeinen Formel V

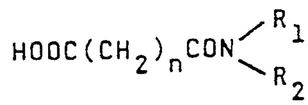

. (V)

worin n, $R_1$ und $R_2$ die oben genannte Bedeutung besitzen, oder ein reaktionsfähiges Derivat dieser Carbonsäure, mit einem Amin der allgemeinen Formel VI

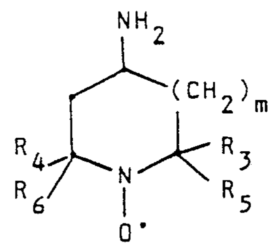

(VI)

worin m, $R_3$, $R_4$, $R_5$ und $R_6$ die obengenannte Bedeutung besitzen, kondensiert und gewünschtenfalls vorhandene Ketalgruppen oder Acylgruppen hydrolytisch spaltet.

Die neuen Verbindungen der allgemeinen Formel I mit B in der Bedeutung eines Protein-, Zucker- oder Lipidrestes werden nach einem Verfahren hergestellt, indem man in an sich bekannter Weise eine Carbonsäure der allgemeinen Formel III, worin ......., m, X, $R_3$, $R_4$, $R_5$ und $R_6$ die oben genannte Bedeutung besitzen, oder ein reaktionsfähiges Derivat dieser Carbonsäure, mit einer nucleophilen Gruppe eines Proteins, Zuckers oder Lipids umsetzt.

Die Herstellung der Nitroxyle der allgemeinen Formel I mit B in der Bedeutung einer $—NR_1R_2$-Gruppe aus den Verbindungen der allgemeinen Formel II erfolgt unter Bedingungen, wie sie dem Fachmann wohlbekannt sind. So kann man die Verbindungen der allgemeinen Formel II beispielsweise mit Wasserstoffperoxid in Gegenwart von Wolframatkatalysatoren, wie zum Beispiel Natriumwolframat oxydieren. Besonders gut gelingt die Oxidation mit organischen Persäuren wie Perbenzoesäure, 3-Chlorperbenzoesäure und Peressigsäure.

Die Bildung der Amide aus den entsprechenden Carbonsäuren und Aminen erfolgt ebenfalls in konventioneller Weise. (Houben-Weyl, Methoden der Organischen Chemie, Band 15/2, 1974, Seite 1 ff.) So kann man beispielsweise die Carbonsäuren in das entsprechende Säurechlorid oder gemischte Anhydrid (z. B. mit Trifluoressigsäureanhydrid oder Chlorameisensäuremethylester) überführen und dieses mit dem Amin umsetzen. Ferner ist es auch möglich, beide Komponenten in Gegenwart eines wasserentziehenden Mittels (wie N,N'-Carbonyldiimidazol oder Dicyclohexylcarbodiimid) zu kondensieren.

Die sich gegebenenfalls anschließende Abspaltung der Schutzgruppen erfolgt ebenfalls in konventioneller Weise. Genannt sei die Ketalspaltung mittels Säuren und die Esterspaltung mittels alkoholischer Säuren oder Basen.

Die neuen Hydroxyalkylamide sind auch besonders wegen ihrer hohen in-vivo-Stabilität bezüglich ihrer Nitroxylfunktion wertvolle Diagnostika.

Die Bildung der Konjugate mit Biomolekülen erfolgt ebenfalls nach an sich bekannten Methoden beispielsweise durch Reaktion der nucleophilen Gruppe eines Biomoleküls, wie der Amino-, Phenol-, Sulfhydryl- oder Imidazolgruppe mit einem aktivierten Derivat der allgemeinen Formel III. Als aktivierte Derivate kommen beispielsweise Säurechloride, gemischte Anhydride (siehe zum Beispiel G.E. Krejcarek u. K.L. Tucker, Biochem, Biophys. Res. Commun. 1977, 581), aktivierte Ester, Nitrene oder Isothiocyanate in Betracht.

Die Ausgangsverbindungen für das erfindungsgemäße Verfahren sind bekannt oder können in an sich bekannter Weise hergestellt werden. Es wurde gefunden, daß die Synthese der bekannten Verbindungen oft auf einem wesentlich einfacheren Weg durchgeführt werden kann, als in der Literatur beschrieben, wie die nachfolgenden Herstellungsvorschriften zeigen.

2,2,5,5-Tetramethyl-pyrrolin-3-carbonsäuremethylester.

Zu 1 200 ml Natriummethylatlösung in der 20,69 g (900 m mol) Natrium enthalten sind, werden unter Rühren und Kühlung bei 10 bis 15 °C anteilweise 118,19 g (300 m mol) 3,5-Dibrom-2,2,6,6-tetramethylpi-

peridin-4-on Hydrobromid gegeben. Nach 30 Minuten Rühren engt man im Vakuum ein, nimmt den Rückstand in trockenem Diethylether auf, filtriert vom Feststoff ab, engt die Lösung erneut im Vakuum ein und destilliert den Rückstand im Wasserstrahlvakuum.

Der 2,2,5,5-Tetramethyl-3-pyrrolin-3-carbonsäuremethylester geht bei 88 bis 89/15 mm als farblose Flüssigkeit über. Man erhält 54,2 g (91 % der Theorie).

Die Verbindung ist gemäß Gaschromatogramm 92,3 %ig, sie ist für die Weiterverarbeitung genügend rein.

2,2,5,5-Tetramethyl-3-pyrrolin-1-oxyl-3-carbonsäuremethylester.

In 70 ml Dichlormethan werden 13,38 g (73 m mol) 2,2,5,5-Tetramethyl-3-pyrrolin-3-carbonsäuremethylester gelöst, mit 0,1 g (1,2 m mol) Natriumacetat versetzt, auf — 10 °C gekühlt und unter Rühren mit der gekühlten Lösung von 30 g (146 m mol) 37 %iger Peressigsäure, die weitere 0,8 g (9,6 m mol) Natriumacetat enthält anteilweise versetzt. Nach beendeter Zugabe wird 4 Stunden nachgerührt. Dann verdünnt man mit Dichlormethan und rührt in Natriumbikarbonatlösung ein um die Essigsäure zu neutralisieren. Die organische Phase wird abgetrennt, über Natriumsulfat getrocknet und im Vakuum zur Trockene eingeengt. Der 2,2,5,5-Tetramethyl-3-pyrrolin-1-oxyl-3-carbonsäuremethylester kristallisiert aus Diethylether in Nadeln vom Schmelzpunkt 87 bis 89 °C.

Die Ausbeute beträgt 12,63 g (87,3 % der Theorie).

2,2,5,5-Tetramethyl-3-pyrrolin-1-oxyl-3-carbonsäure.

Zu einer Lösung von 15,10 g (76,17 m mol) 2,2,5,5-Tetramethyl-3-pyrrolin-1-oxyl-3-carbonsäuremethylester in 40 ml destilliertem Wasser und 10 ml Ethanol wurden 4,57 g (114,26 m mol) Natriumhydroxid in 40 ml destilliertem Wasser gegeben. Man erwärmt 1,5 Stunden unter Rühren auf 60 °C, verdünnt mit 200 ml Wasser, säuert mit 4 N Schwefelsäure auf einen pH-Wert von 3 an und extrahiert die Säure mit Methylenchlorid. Die organische Phase wird über Natriumsulfat getrocknet und im Vakuum zur Trockne eingeengt. Die 2,2,5,5-Tetramethyl-3-pyrrolin-1-oxyl-3-carbonsäure kristallisiert aus Ethanol/Diethylether (Schmelzpunkt 221 bis 223 °C). Die Ausbeute beträgt 13,82 g (98,5 % der Theorie).

2,2,5,5-Tetramethylpyrrolidin-3-carbonsäuremethylester.

87,80 g (479 m mol) 2,2,5,5-Tetramethyl-3-pyrrolin-3-carbonsäuremethylester werden in 1 000 ml Methanol gelöst und in Gegenwart von 11 g Raney-Nickel und einem Anfangsdruck von 180 bar bei Raumtemperatur hydriert. Man saugt vom Katalysator ab, behandelt die Lösung mit Aktivkohle, engt im Vakuum ein und destilliert den Rückstand im Wasserstrahlvakuum. Der 2,2,5,5-Tetramethylpyrrolidin-3-carbonsäuremethylester geht bei 86 bis 87°/14 mm über. Man erhält 77,1 g (88 % der Theorie). Die Verbindung ist gemäß Gaschromatogramm 98,7 %ig.

| $C_{10}H_{19}NO_2$ | (185,27) | | | |
|---|---|---|---|---|
| Ber. : | 64,83 | C 10,34 | H 7,56 | N |
| Gef. : | 64,71 | C 10,44 | H 7,44 | N |

2,2,5,5-Tetramethylpyrrolidin-1-oxyl-3-carbonsäuremethylester.

Zu einer Lösung von 20,30 g (108,15 m mol) 2,2,5,5-Tetramethylpyrrolidin-3-carbonsäuremethylester (98,7 %ig) in 200 ml Dichlormethan werden 0,14 g (1,6 m mol) Natriumacetat gegeben, und die Lösung wird auf — 10 °C abgekühlt. Zu der gerührten Lösung werden 45,2 g (220 m mol) Peressigsäure, (37 %ig, 1 % Schwefelsäure), ebenfalls gekühlt, sowie 1,22 g (14,7 m mol) Natriumacetat, in der Peressigsäure suspendiert, getropft. Man läßt über Nacht rühren, neutralisiert die Essigsäure mit Natriumbikarbonat, trennt die organische Phase ab, trocknet sie über Natriumsulfat und engt sie im Vakuum ein. Der Rückstand wird an der Ölpumpe destilliert. Bei 76°/0,01 torr gehen 20,23 g (93,4 % der Theorie) eines orange gefärbten Öles über.

| $C_{10}H_{18}NO_3$ | (200,26) | | | |
|---|---|---|---|---|
| Ber. : | 59,98 | C 9,06 | H 6,99 | N |
| Gef. : | 59,89 | C 9,17 | H 6,93 | N |

2,2,5,5-Tetramethylpyrrolidin-1-oxyl-3-carbonsäure.

In 120 ml destilliertem Wasser werden 8,75 g (218,87 m mol) Natriumhydroxid gelöst und mit der Lösung von 29,22 g (145,91 m mol) 2,2,5,5-Tetramethylpyrrolidin-1-oxyl-3-carbonsäuremethylester in 20 ml Ethanol versetzt. Man erwärmt unter Rühren 90 Minuten auf 60 °C, verdünnt mit 200 ml Wasser und säuert mit 4 N Schwefelsäure auf einen pH-Wert von 3 an. Die Säure wird in Dichlormethan aufgenommen, die organische Lösung über Natriumsulfat getrocknet und im Vakuum zur Trockene eingeengt. Der Rückstand wird aus Dichlormethan/Diethylether kristallisiert. Man erhält so 26,12 g (96,1 % der Theorie) 2,2,5,5-Tetramethyl-pyrrolidin-1-oxyl-3-carbonsäure vom Schmelzpunkt 197 bis 199 °C.

| $C_9H_{16}NO_3$ | (186,23) | | | |
|---|---|---|---|---|
| Ber. : | 58,05 | C 8,66 | H 7,52 | N |
| Gef. : | 58,11 | C 8,72 | H 7,35 | N |

Bernsteinsäure-(2,2,6,6-tetramethyl-1-oxyl-4-piperidyl)-monoamid.

55 g (= 321 m mol) 4-Amino-2,2,6,6-tetramethylpiperidin-1-oxyl werden in 55 ml Pyridin gelöst. Hierzu tropft man unter Rühren eine Lösung von 40,7 g (407 m mol) Bernsteinsäureanhydrid in 500 ml Tetrahydrofuran. Man läßt 12 Stunden bei Raumtemperatur nachrühren und engt im Vakuum zur Trockne ein. Der Rückstand wird mit 1 000 ml Wasser versetzt und die wässrige Lösung zur Entfernung des Pyridins mit Ether extrahiert. Dann engt man im Vakuum auf ca. 200 ml ein und saugt den gebildeten Niederschlag nach mehrstündigem Rühren im Eisbad ab. Nach Waschen mit wenig eiskaltem Wasser und Trocknen bei 40 °C im Vakuum erhält man 81,3 g (= 93 % der Theorie) als orangefarbene Kristalle vom Schmelzpunkt 108 bis 110 °C.

| | | | | |
|---|---|---|---|---|
| $C_{13}H_{23}N_2O_4$ | (271,34) | | | |
| Ber. : | 57,54 | C 8,54 | H 10,33 | N |
| Gef. : | 57,48 | C 8,60 | H 10,41 | N |

Die erfindungsgemäßen Verbindungen der Formel I sind wertvolle Diagnostika. Zur praktischen Anwendung werden sie in Wasser oder physiologischer Salzlösung gelöst oder suspendiert und gegebenenfalls mit den in der Galenik üblichen Zusätzen in eine für die intravasale oder orale Applikation geeignete Form überführt, so daß ihre Konzentrationen in einem Bereich von 1 µMol/l bis 1 Mol/l liegen.

Die Verträglichkeit der neuen diagnostischen Mittel ist denen der bekannten vergleichbaren Verbindungen eindeutig überlegen. Toxische Wirkungen der bisher zur Salzbildung benötigten Ionen wie Natrium, Kalium, Meglumin usw. entfallen völlig oder größtenteils. Der osmotische Druck der konzentrierten Lösungen dieser diagnostischen Mittel ist drastisch reduziert. Auch dadurch wird die Verträglichkeit nach oraler und parenteraler Gabe wesentlich verbessert, da stark hypertone Lösungen Blutgefäße und Gewebe schädigen, Herz und Kreislauf beeinflussen und unerwünschte diuretische Wirkungen entfalten.

Die erfindungsgemäßen Verbindungen beeinflussen in günstiger Weise die Relaxationszeiten der Protonen. Sie weisen im Vergleich zu den bekannten Nitroxylradikalen eine bessere Bioverfügbarkeit auf und sind — wie folgende Versuche zeigen — wesentlich weniger toxisch als diese :

Mäusen im Gewicht von 18 bis 22 g werden unterschiedliche Mengen Substanz als 0,5 m mol wässrige Lösung pro kg, eingestellt auf pH 7 bis 7,5 — intravenös in die Schwanzvene injiziert. Nach 7 Tagen wird die Überlebensrate der Mäuse ermittelt und aus dieser in üblicher Weise die $LD_{50}$ in m mol/kg Tier ermittelt.

Die nachfolgende Tabelle zeigt die hierbei erhaltenen Ergebnisse im Vergleich zu den vorbekannten Verbindungen 1 und 2. (J. Comput. Assist. Tomogr. 7, 1983, 184).

| Nr. | Substanz | $LD_{50}$ in m mol/kg Maus |
|---|---|---|
| 1 | Bernsteinsäure-(2,2,6,6-tetramethyl-1-oxyl-piperidin-4-yl)-amid | 10 |
| 2 | 2,2,5,5-Tetramethyl-pyrrolidin-1-oxyl-3-carbonsäure | 10 |
| 3 | 2,2,5,5-Tetramethyl-3-pyrrolin-1-oxyl-3-carbonsäure-(1,3,4-trihydroxybut-2-yl)-amid | 20 |
| 4 | 2,2,5,5-Tetramethyl-3-pyrrolin-1-oxyl-3-carbonsäure-(2,3-dihydroxypropyl)-amid | 15 |
| 5 | 2,2,5,5-Tetramethylpyrrolidin-1-oxyl-3-carbonsäure-(1,3,4-trihydroxybut-2-yl)-amid | 25 |

Insgesamt ist es damit gelungen, neue diagnostische Mittel bereitzustellen, die neue Möglichkeiten in der diagnostischen Medizin erschließen. Vor allem die Entwicklung neuartiger bildgebender Verfahren in der medizinischen Diagnostik läßt diese Entwicklung als äußerst wünschenswert und notwendig erscheinen.

Die nachfolgenden Ausführungsbeispiele dienen zur näheren Erläuterung des erfindungsgemäßen Verfahrens und der Verwendung der Verfahrensprodukte.

Beispiel 1

2,2,5,5-Tetramethylpyrrolidin-1-oxyl-3-carbonsäure-[2-hydroxy-2-(2,2-dimethyl-1,3-dioxolan-4-yl)-ethyl]-amid.

Zu einer Lösung von 14,82 g (79,58 m mol) 2,2,5,5-Tetramethylpyrrolidin-1-oxyl-3-carbonsäure in 200 ml absolutem Tetrahydrofuran werden unter Rühren, Kühlung und Abdeckung mit Argon 8,053 g (79,58 m mol) Triethylamin gegeben. Nach Abkühlen auf —5° werden innerhalb 45 Minuten 8,638 g (79,59 m mol) Chlorameisensäureethylester in 10 ml absolutem Tetrahydrofuran unter Rühren zugetropft. Es wird 30 Minuten bei —5° gerührt. Dann werden 12,83 g (79,59 m mol) 2-Amino-1-(2,2-dimethyl-1,3-dioxolan-4-yl)-ethanol anteilweise zugegeben. Nach 30 Minuten wird die Kühlung entfernt und es wird 3 Stunden bei Raumtemperatur gerührt. Dann wird mit 200 ml absolutem Ether verdünnt, vom Feststoff abgesaugt, der mit absolutem Ether gewaschen wird. Die organische Lösung wird im Vakuum zur Trockene eingeengt. Der Rückstand wird in Dichlormethan aufgenommen, mit halbgesättigter Natriumbikarbonatlösung gewaschen, über Natriumsulfat getrocknet und im Vakuum zur Trockene eingeengt. Das verbleibende gelbe Öl kristallisiert nach Zusatz von Diethylether. Das 2,2,5,5-Tetramethylpyrrolidin-1-oxyl-3-carbonsäure-[2-hydroxy-2-(2,2-dimethyl-1,3-dioxolan-4-yl)-ethyl]-amid schmilzt nach Umkristallisation aus Essigester bei 144°, und man erhält 14,79 g (56,4 % der Theorie) der Verbindung. Aus der Bikarbonatlösung werden 4,0 g Ausgangssäure zurückgewonnen.

## Beispiel 2

2,2,5,5-Tetramethylpyrrolidin-1-oxyl-3-carbonsäure-(2,3,4-trihydroxybutyl)-amid

In 150 ml Wasser, in dem 0,1 ml (3,6 m mol) konzentrierte Schwefelsäure enthalten sind, werden 13,9 g (42,46 mol) 2,2,5,5-Tetramethylpyrrolidin-1-oxyl-3-carbonsäure-[2-hydroxy-2-(2,2-dimethyl-1,3-dioxolan-4-yl)-ethyl]-amid suspendiert. Unter Rühren wird 3 Stunden auf 40-50 °C erwärmt. Die Lösung wird abgekühlt, und die Schwefelsäure mit Ionenaustauscher Amberlite® IRA 410 OH—-Form neutralisiert. Der Austauscher wird abfiltriert und mit Wasser gewaschen. Die vereinigten Lösungen werden im Vakuum zur Trockene eingeengt. Das 2,2,5,5-Tetramethylpyrrolidin-1-oxyl-3-(2,3,4-trihydroxybutyl)-amid wird aus Ethanol kristallisiert, und man erhält 10,97 g (89,3 % der Theorie) gelbe Kristalle vom Schmelzpunkt 172 °C. Durch Umkristallisation aus Wasser erhält man gelbe Nadeln vom Schmelzpunkt 189-91 °C.

## Beispiel 3

2,2,5,5-Tetramethylpyrrolidin-1-oxyl-3-carbonsäure-[N-(2,3,4,5,6-pentahydroxyhexyl)-N-methyl]-amid

Zu einer Lösung von 19,93 g (107 m mol) 2,2,5,5-Tetramethylpyrrolidin-1-oxyl-3-carbonsäure in 300 ml Dimethylformamid werden unter Rühren, Kühlung und Abdeckung mit Argon 12,05 g (119 m mol) Triethylamin gegeben. Nach Abkühlen auf —10 °C werden innerhalb 30 Minuten 12,92 g (119 m mol) Chlorameisensäureethylester in 30 ml absolutem Tetrahydrofuran gelöst gegeben. Nach 10 Minuten werden 10,89 g (107 m mol) N-Methylglucamin anteilweise zugegeben. Man läßt 2 Stunden bei 0 °C rühren, entfernt die Kühlung und rührt über Nacht bei Raumtemperatur. Es wird vom Feststoff abfiltriert, der mit Dimethylformamid und absolutem Tetrahydrofuran gewaschen wird. Die vereinigten Lösungen werden im Vakuum, zuletzt an der Ölpumpe, eingeengt. Der Rückstand wird in absolutem Tetrahydrofuran aufgenommen und aus Diethylether umgefüllt. Das ausgefallene Öl wird in Wasser gelöst und durch Behandeln mit Ionenaustauscher Amberlite® IR 120 H°-Form und IRA 410 OH°-Form ionenfrei erhalten. Die gelbe Lösung wird im Vakuum zur Trockene eingeengt. Der verbleibende Sirup wird im Vakuum über Diphosphorpentoxid aufbewahrt, da er stark hygroskopisch ist. Es werden 24,8 g (63,8 % der Theorie) 2,2,5,5-Tetramethylpyrrolidin-1-oxyl-carbonsäure-[N-(2,3,4,5,6-pentahydroxyhexyl)-N-methyl]-amid als gelb-oranger Sirup erhalten.

| $C_{16}H_{31}N_2O_7$ | MG 363,43 | | |
|---|---|---|---|
| Ber.: | 52,88 C | 8,60 H | 7,71 N |
| Gef.: | 52,61 C | 8,78 H | 7,59 N |

## Beispiel 4

2,2,5,5-Tetramethylpyrrolidin-1-oxyl-3-carbonsäure-(2,3-dihydroxypropyl)-amid

Zu einer Lösung von 13,61 g (73,1 m mol) 2,2,5,5-Tetramethylpyrrolidin-1-oxyl-3-carbonsäure in 150 ml Dichlormethan werden unter Rühren, Kühlung und Abdeckung mit Argon 8,13 g (80,3 m mol) Triethylamin gegeben. Nach Abkühlen auf —10 °C werden 8,72 g (80,3 m mol) Chlorameisensäureethylester in 10 ml Dichlormethan zugetropft, und nach 20 Minuten Rühren bei —5 °C wurden 6,66 g (73,1 m mol) 2,3-Dihydroxypropylamin in 40 ml Dimethylacetamid zugetropft. Man entfernt die Kühlung und läßt eine Stunde nachrühren, saugt vom Feststoff ab, wäscht mit absolutem Tetrahydrofuran nach und engt im Vakuum, zuletzt an der Ölpumpe, zur Trockene ein. Der Rückstand wird mit absolutem Ether ausgezogen, im Wasser gelöst und durch Behandeln mit Ionenaustauscher Amberlite® IR 120 $H^+$-Form und IRA 410 OH—-Form ionenfrei erhalten. Die gelbe Lösung wird im Vakuum zur Trockene eingeengt, und man erhält 12,4 g (65,5 % der Theorie) 2,2,5,5-Tetramethylpyrrolidin-1-oxyl-3-carbonsäure-(2,3-dihydroxypropyl)-amid als gelb-orangen Sirup.

| | | | | |
|---|---|---|---|---|
| $C_{12}H_{23}N_2O_4$ | MG 259,33 | | | |
| Ber.: | 55,58 | C 8,94 | H 10,80 | N |
| Gef.: | 55,33 | C 9,19 | H 10,62 | N |

Beispiel 5

2,2,5,5-Tetramethylpyrrolidin-1-oxyl-3-carbonsäure-bis (2-hydroxyethyl)-amid

Zu einer Lösung von 12,91 g (69,32 m mol) 2,2,5,5-Tetramethylpyrrolidin-1-oxyl-3-carbonsäure in 150 ml absolutem Tetrahydrofuran werden unter Rühren, Kühlung und Abdeckung mit Argon 7,69 g (76,0 m mol) Triethylamin gegeben. Nach Abkühlen auf —10 °C werden 8,25 g (76,0 m mol) Chlorameisensäureethylester in 10 ml absolutem Tetrahydrofuran zugetropft, und nach 20 Minuten Rühren bei —5 °C werden 7,2 g (69,32 m mol) Diethanolamin in 40 ml Dioxan zugetropft. Man entfernt die Kühlung und läßt 1 Stunde nachrühren. Dann wird mit 200 ml absolutem Ether verdünnt, vom Feststoff abgesaugt, mit Ether nachgewaschen und im Vakuum eingeengt. Der Rückstand wird in Wasser aufgenommen und durch Behandeln mit Ionenaustauscher Amberlite® IR 120 $H^+$-Form und IRA 410 $OH^-$-Form ionenfrei erhalten. Die gelbe neutrale Lösung wird im Vakuum zur Trockene eingeengt und man erhält 11,64 g (61,4 % der Theorie) 2,2,5,5-Tetramethylpyrrolidin-1-oxyl-3-carbonsäure-bis(2-hydroxyethyl)-amid als gelb-orangen Sirup.

| | | | | |
|---|---|---|---|---|
| $C_{13}H_{25}N_2O_4$ | MG 273,36 | | | |
| Ber.: | 57,12 | C 9,22 | H 10,25 | N |
| Gef.: | 57,01 | C 9,38 | H 10,10 | N |

Beispiel 6

2,2,5,5-Tetramethylpyrrolidin-1-oxyl-3-carbonsäure-(1,3-dihydroxyprop-2-yl)-amid

Zu einer Lösung aus 11,41 g (61,27 m mol) 2,2,5,5-Tetramethylpyrrolidin-1-oxyl-3-carbonsäure und 6,20 g (61,27 m mol) Triethylamin in 200 ml absolutem Tetrahydrofuran werden bei —5 °C unter Rühren und Abdeckung mit Argon 6,65 g (61,27 m mol) Chlorameisensäureethylester in 10 ml Tetrahydrofuran getropft. Nach 30 Minuten werden anteilweise 5,59 g (61,3 m mol) gepulvertes 2-Amino-1,3-propandiol zugegeben. Man entfernt das Kältebad und läßt 5 Stunden nachrühren. Dann wird mit 200 ml absolutem Diethylether verdünnt, vom Feststoff abgesaugt, mit Ether nachgewaschen und im Vakuum eingeengt. Der Rückstand wird in Wasser aufgenommen und durch Behandeln mit Ionenaustauscher Amberlite® IR 120 $H^+$-Form und IRA 410 $OH^-$-Form ionenfrei erhalten. Die neutrale gelbe Lösung wird im Vakuum zur Trockene eingeengt und man erhält 10,1 g (64,4 % der Theorie) 2,2,5,5-Tetramethylpyrrolidin-1-oxyl-3-carbonsäure-(1,3-dihydroxyprop-2-yl)-amid als dunkelgelben Sirup.

| | | | | |
|---|---|---|---|---|
| $C_{12}H_{23}N_2O_4$ | (259,33) | | | |
| Ber.: | 55,58 | C 8,94 | H 10,80 | N |
| Gef.: | 55,37 | C 9,19 | H 10,64 | N |

Beispiel 7

2,2,5,5-Tetramethyl-3-pyrrolin-3-carbonsäure-(2,2-dimethyl-1,3-dioxolan-4-ylmethyl)-amid

Zu einer Lösung aus 22,93 g (175 m mol) (2,2-Dimethyl-1,3-dioxolan-4-yl)-methylamin und 58,9 g (525 m mol) Kalium-tert-butylat in 700 ml Isopropanol werden unter Rühren, Kühlung zwischen —5 bis —0 °C und Argonabdeckung anteilweise 68,94 g (175 m mol) 3,5-Dibrom-2,2,6,6-tetramethyl-piperidin-4-on Hydrobromid gegeben. Es bildet sich ein weißer Brei. Man läßt 1 Stunde ohne Kühlung nachrühren, saugt vom Feststoff ab und engt im Vakuum zur Trockene ein. Der Rückstand wird zwischen Dichlormethan und Wasser verteilt. Die organische Phase wird über Natriumsulfat getrocknet und im Vakuum zur Trockene eingeengt. Der Rückstand kristallisiert. Nach Umkristallisation aus Pentan erhält man 30,1 g (60,9 % der Theorie) 2,2,5,5-Tetramethyl-3-pyrrolin-3-carbonsäure-(2,2-dimethyl-1,3-dioxlan-4-ylmethyl)-amid vom Schmelzpunkt 55-57 °C.

| | | | | |
|---|---|---|---|---|
| $C_{15}H_{26}N_2O_3$ | (282,40) | | | |
| Ber.: | 63,35 | C 9,92 | H 9,82 | N |
| Gef.: | 63,11 | C 9,72 | H 9,99 | N |

Beispiel 8

2,2,5,5-Tetramethyl-3-pyrrolin-1-oxyl-3-carbonsäure-(2,2-dimethyl-1,3 dioxolan-4-ylmethyl)-amid

In 200 ml absolutem Diethylether werden 15,88 g (56,23 m mol) 2,2,5,5-Tetramethyl-3-pyrrolin-3-carbonsäure-(2,2-dimethyl-1,3-dioxolan-4-ylmethyl)-amid gelöst, auf —5 °C gekühlt und mit der Lösung von 23,92 g (112,4 m mol) 3-Chlorperbenzoesäure, 80 %, in 150 ml absolutem Ether anteilweise unter Rühren versetzt. Man entfernt das Kältebad, läßt 2 Stunden nachrühren, rührt in 200 ml Sodalösung in der

29 g Natriumkarbonat-Dekahydrat enthalten sind, und nimmt das Produkt in Diethylether auf. Die Etherlösung wird über Natriumsulfat getrocknet und im Vakuum zu Trockene eingeengt. Man erhält so 16,46 g (98,4 % der Theorie) 2,2,5,5-Tetramethyl-3-pyrrolin-1-oxyl-3-carbonsäure-(2,2-dimethyl-1,3-dioxolan-4-ylmethyl)-amid als orange gefärbten Sirup.

$C_{15}H_{25}N_2O_4$ (297,38)
Ber.: 60,59 C 8,47 H 9,42 N
Gef.: 60,33 C 8,68 H 9,30 N

Beispiel 9

2,2,5,5-Tetramethyl-3-pyrrolin-1-oxyl-3-carbonsäure-(2,3-dihydroxypropyl)-amid

14,2 g (47,8 m mol) 2,2,5,5-Tetramethyl-3-pyrrolin-1-oxyl-3-carbonsäure-(2,2-dimethyl-1,3-dioxolan-4-ylmethyl)-amid werden in 100 ml destilliertem Wasser, in dem 0,1 ml (3,6 m mol) konzentrierte Schwefelsäure enthalten sind, suspendiert und 3 Stunden bei 50 °C gerührt. Nach dem Abkühlen wird mit Ionenaustauscher Amberlite® IRA 410 OH$^-$-Form neutralisiert. Man wäscht das Harz mit Wasser und engt die vereinigten Lösungen im Vakuum ein. Der Rückstand wird an der Ölpumpe bei Raumtemperatur getrocknet. Man erhält so 10,48 g (85,3 % der Theorie) kristallines 2,2,5,5-Tetramethyl-3-pyrrolin-1-oxyl-3-carbonsäure-(2,3-dihydroxypropyl)-amid mit einem Schmelzpunkt von 131 bis 133 °C.

$C_{12}H_{21}N_2O_4$ (257,31)
Ber.: 56,02 C 8,23 H 10,89 N
Gef.: 55,87 C 8,39 H 10,74 N

Beispiel 10

2,2,5,5-Tetramethyl-3-pyrrolin-3-carbonsäure-[N-(2,2-dimethyl-1,3-dioxolan-4-ylmethyl)-N-methyl]-amid

In Analogie zu Beispiel 7 werden umgesetzt: 68,94 g (175 m mol) 3,5-Dibrom-2,2,6,6-tetramethylpiperidin-4-on Hydrobromid, 58 g (525 m mol) Kalium-tert-butylat und 25,41 g (115 m mol) N-Methyl-N-(2,2-dimethyl-1,3-dioxolan-4-ylmethyl)-amin in 600 ml Isopropanol.

Man erhält so 39,43 g (76 % der Theorie) 2,2,5,5-Tetramethyl-3-pyrrolin-3-carbonsäure-[N-(2,2-dimethyl-1,3-dioxolan-4-ylmethyl)-N-methyl]-amid als orange gefärbten Sirup.

$C_{10}H_{28}N_2O_3$ (296,41)
Ber.: 64,84 C 9,52 H 9,45 N
Gef.: 64,59 C 9,73 H 9,27 N

Beispiel 11

2,2,5,5-Tetramethyl-3-pyrrolin-1-oxyl-3-carbonsäure-[N-[(2,2-dimethyl-1,3-dioxolan-4-ylmethyl)-N-methyl]-amid

In Analogie zu Beispiel 8 werden umgesetzt:

14,97 g (50,50 m mol) 2,2,5,5-Tetramethyl-3-pyrrolin-3-carbonsäure-N-[(2,2-dimethyl-1,3-dioxolan-4-yl)-methyl]-N-methyl-amid mit 22,0 g (101 m mol) 3-Chlorperbenzoesäure 80 % in 300 ml absoluten Diethylether. Man erhält so 13,12 g (83,5 % der Theorie) 2,2,5,5-Tetramethyl-3-pyrrolin-1-oxyl-3-carbonsäure-[N-(2,2-dimethyl-1,3-dioxolan-4-ylmethyl]-N-methyl]-amid, das nach Kristallisation aus Essigester einen Schmelzpunkt von 69 bis 71 °C zeigt.

$C_{16}H_{28}N_2O_3$ (296,41)
Ber.: 61,51 C 9,03 H 8,97 N
Gef.: 61,80 C 8,98 H 8,87 N

Beispiel 12

2,2,5,5-Tetramethyl-3-pyrrolin-1-oxyl-3-carbonsäure-N-(2,3-dihydroxypropyl)-N-methyl-amid

In Analogie zu Beispiel 9 werden 11,49 g (36,9 m mol) 2,2,5,5-Tetramethyl-3-pyrrolin-1-oxyl-3-carbonsäure-[N-(2,2-dimethyl-1,3-dixolan-4-ylmethyl)-N-methyl]-amid in 100 ml destilliertem Wasser, in dem 0,05 ml (1,8 m mol) konzentrierte Schwefelsäure enthalten sind, verseift. Man erhält so 9,18 g (91,7 % der Theorie) 2,2,5,5-Tetramethyl-3-pyrrolin-1-oxyl-3-carbonsäure-N-(2,3-dihydroxypropyl)-N-methyl-amid als orange gefärbten Sirup.

$C_{13}H_{23}N_2O_4$ (271,34)
Ber.: 57,55 C 8,54 H 10,32 N
Gef.: 57,33 C 8,75 H 10,18 N

Beispiel 13

2,2,5,5-Tetramethylpyrrolidin-3-carbonsäure-N-[(2,2-dimethyl-1,3-dioxolan-4-ylmethyl)-N-methyl]-amid

Zu einer Lösung von 22,58 g (76,18 m mol) 2,2,5,5-Tetramethyl-3-pyrrolin-3-carbonsäure-[(2,2-dimethyl-1,3-dioxolan-4-ylmethyl]-amid in 400 ml Methanol werden 2 g Raney-Nickel B 115 Z geben, und es wird bei einem Anfangsdruck von 180 Bar hydriert. Es wird genau 1 Mol Wasserstoff aufgenommen. Man saugt vom Katalysator ab, behandelt die Lösung mit Kohle und engt im Vakuum zur Trockene ein. Man erhält so 21,0 g (92,4 % der Theorie) 2,2,5,5-Tetramethylpyrrolidin-3-carbonsäure-[N-(2,2-dimethyl-1,3-dioxolan-4-ylmethyl]-N-methyl]-amid als blaßgelbes Öl.

$C_{16}H_{30}N_2O_3$ (298,43)

| | | | |
|---|---|---|---|
| Ber. : | 64,40 C | 10,13 H | 9,39 N |
| Gef. : | 64,66 C | 10,21 H | 9,57 N |

Beispiel 14

2,2,5,5-Tetramethylpyrrolidin-1-oxyl-3-carbonsäure-[N-(2,2-dimethyl-1,3-dioxolan-4-ylmethyl)-N-methyl]-amid

In Analogie zu Beispiel 8 werden umgesetzt :

20,78 g (69,63 m mol) 2,2,5,5-Tetramethylpyrrolidin-3-carbonsäure-N-[(2,2-dimethyl-1,3-dioxolan-4-ylmethyl)-N-methyl]-amid mit 30 g (140 m mol) 3-Chlorperbenzoesaüre, 80 %, in 400 ml absolutem Diethylether. Man erhält 17,52 g (80,3 % der Theorie) 2,2,5,5-Tetramethylpyrrolidin-1-oxyl-3-carbonsäure-N-[(2,2-dimethyl-1,3-dioxolan-4-ylmethyl)-N-methyl]-amid als orange gefärbten Sirup.

$C_{16}H_{29}N_2O_4$ (313,42)

| | | | |
|---|---|---|---|
| Ber. : | 61,32 C | 9,33 H | 8,94 N |
| Gef. : | 61,61 C | 9,40 H | 8,83 N |

Beispiel 15

2,2,5,5-Tetramethylpyrrolidin-1-oxyl-3-carbonsäure-N-(2,3-dihydroxypropyl)-N-methyl-amid

In Analogie zu Beispiel 9 werden 16,60 g (53 m mol) 2,2,5,5-Tetramethylpyrrolidin-1-oxyl-3-carbonsäure-[N-(2,2-dimethyl-1,3-dioxolan-4-ylmethyl)-N-methyl]-amid in 150 ml destilliertem Wasser, in dem 0,05 ml (1,8 m mol) konzentrierte Schwefelsäure gelöst wird, verseift. Man erhält so 13,28 g (91,7 % der Theorie) 2,2,5,5-Tetramethylpyrrolidin-1-oxyl-3-carbonsäure-N-(2,3-dihydroxypropyl)-N-methyl-amid als orange gefärbten Sirup.

$C_{13}H_{25}N_2O_4$ (273,36)

| | | | |
|---|---|---|---|
| Ber. : | 57,12 C | 9,22 H | 10,25 N |
| Gef. : | 56,86 C | 9,39 H | 10,06 N |

Beispiel 16

2,2,5,5-Tetramethyl-3-pyrrolin-3-carbonsäure-[2-hydroxy-2-(2,2-dimethyl-1,3-dioxolan-4-yl)-ethyl]-amid

In Analogie zu Beispiel 7 werden umgesetzt :

51,22 g (130 m mol) 3,5-Dibrom-2,2,6,6-Tetramethylpiperidin-4-on Hydrobromid 45,05 g (390 m mol) Kalium-tert-butylat und 20,96 g (130 m mol) 2-Amino-1-(2,2-dimethyl-1,3-dicxolan-4-yl)-ethanol in 500 ml Isopropanol. Man erhält so 22,93 g (56,5 % der Theorie) 2,2,5,5-Tetramethyl-3-pyrrolin-3-carbonsäure-[2-hydroxy-2-(2,2-dimethyl-1,3-dioxolan-4-yl)-ethyl]-amid, das aus Diethylether Pentan kristallisiert, einen Schmelzpunkt von 99 bis 101 °C zeigt.

$C_{16}H_{28}N_2O_4$ (312,41)

| | | | |
|---|---|---|---|
| Ber. : | 61,51 C | 9,03 H | 8,97 N |
| Gef. : | 61,57 C | 9,33 H | 8,90 N |

Beispiel 17

2,2,5,5-Tetramethyl-3-pyrrolin-1-oxyl-3-carbonsäure-[2-hydroxy-2-(2,2-dimethyl-1,3-dioxolan-4-yl)-ethyl]-amid

In Analogie zu Beispiel 8 werden umgesetzt :

2,81 g (9 m mol) 2,2,5,5-Tetramethyl-3-pyrrolin-3-carbonsäure-[2-hydroxy-2-(2,2-dimethyl-1,3-dioxolan-4-yl)-ethyl]-amid mit 4,72 g (18 m mol) 3-Chlorperbenzoesäure in 100 ml absolutem Diethylether. Man

erhält so 2,43 g (83,5 % der Theorie) 2,2,5,5-Tetramethyl-3-pyrrolin-1-oxyl-3-carbonsäure-[2-hydroxy-2-(2,2-dimethyl-1,3-dioxolan-4-yl)-ethyl]-amid vom Schmelzpunkt 104 bis 105 °C.

| | | | | | | |
|---|---|---|---|---|---|---|
| $C_{16}H_{27}N_2O_5$ | (327,40) | | | | | |
| Ber.: | 58,70 | C | 8,31 | H | 24,43 | N |
| Gef.: | 58,46 | C | 9,57 | H | 24,28 | N |

Beispiel 18

2,2,5,5-Tetramethyl-3-pyrrolin-1-oxyl-3-carbonsäure-[2-hydroxy-2-(2,2-dimethyl-1,3-dioxolan-4-yl)-ethyl]-amid

15,98 g (51,15 m mol) 2,2,5,5-Tetramethyl-3-pyrrolin-3-carbonsäure-[2-hydroxy-2-(2,2-dimethyl-1,3-dioxolan-4-)-ethyl]-amid werden in 100 ml destilliertem Wasser gelöst und mit 500 mg (1,34 m mol) Titriplex® III, 500 mg (1,52 m mol) Natriumwolframat Dihydrat sowie 10 ml (88,2 mol) 30 % Wasserstoffperoxid versetzt. Man rührt 5 Tage bei Raumtemperatur in einem dunklen Kolben, säuert mit Zitronensäure auf einen pH-Wert von 3 an und extrahiert das Produkt mit Dichlormethan. Die organische Lösung wird mit Wasser gewaschen, über Natriumsulfat getrocknet und im Vakuum zur Trockene eingeengt. Das orange gefärbte Öl wird in Dieethylether aufgenommen und zur Kristallisation angesetzt. Man erhält so 12,79 g (76,4 % der Theorie) 2,2,5,5-Tetramethyl-3-pyrrolin]-oxyl-3-carbonsäure-[2-hydroxy-2-(2,2-dimethyl-1,3-dioxolan-4-yl)-ethyl]-amid zum Schmelzpunkt 105 bis 106 °C.

Beispiel 19

2,2,5,5-Tetramethyl-3-pyrrolin-1-oxyl-3-carbonsäure-(2,3,4-trihydroxybutyl)-amid

In Analogie zu Beispiel 9 werden 15,56 g (47,53 m mol) 2,2,5,5-Tetramethyl-3-pyrrolin-1-oxyl-3-carbonsäure-[2-hydroxy-2-(2,2-dimethyl-1,3-dioxolan-4-yl)-ethyl]-amid in 200 ml destilliertem Wasser, in dem 0,15 ml (5,4 m mol) konzentrierte Schwefelsäure enthalten sind, verseift. Das Produkt kristallisiert aus Wasser. Man erhält 12,87 g (94,2 % der Theorie) 2,2,5,5-Tetramethyl-3-pyrrolin-1-oxyl-3-carbonsäure-(2,3,4-trihydroxybutyl)-amid vom Schmelzpunkt 183 bis 185 °C.

| | | | | | | |
|---|---|---|---|---|---|---|
| $C_{13}H_{23}N_2O_5$ | (287,34) | | | | | |
| Ber.: | 54,34 | C | 8,07 | H | 9,75 | N |
| Gef.: | 54,21 | C | 8,28 | H | 9,58 | N |

Beispiel 20

2,2,5,5-Tetramethyl-3-pyrrolin-3-carbonsäure-(5-hydroxy-2,2-dimethyl-1,3-dioxepan-6-yl)-amid

In Analogie zu Beispiel 7 werden 78,79 g (200 m mol) 3,5-Dibrom-2,2,6,6-Tetramethylpiperidin-4-on Hydrobromid, 69,34 g Kalium-tert-butylat und 35,47 g (220 m mol) 6-Amino-2,2-dimethyl-1,3-dioxepan-5-ol in 800 ml Isopropanol umgesetzt und anschließend mit Dichlormethan extrahiert. Man erhält so 37,66 g (60,3 % der Theorie) 2,2,5,5-Tetramethyl-3-pyrrolin-3-carbonsäure-(5-hydroxy-2,2-dimethyl-1,3-dioxepan-6-yl)-amid, das aus Diethylether/Hexan kristallisiert, einen Schmelzpunkt von 125 bis 127 °C zeigt.

| | | | | | | |
|---|---|---|---|---|---|---|
| $C_{16}H_{28}N_2O_4$ | (312,41) | | | | | |
| Ber.: | 61,51 | C | 9,03 | H | 8,97 | N |
| Gef.: | 61,32 | C | 9,20 | H | 8,83 | N |

Beispiel 21

2,2,5,5-Tetramethyl-3-pyrrolin-1-oxyl-3-carbonsäure-(5-hydroxy-2,2-dimethyl-1,3-dioxepan-6-yl)-amid

In Analogie zu Beispiel 8 werden umgesetzt:

13,0 g (41,61 m mol) 2,2,5,5-Tetramethyl-3-pyrrolin-3-carbonsäure-(5-hydroxy-2,2-dimethyl-1,3-dioxepan-6-yl)-amid mit 19,15 g (90,0 m mol) 3-Chlorperbenzoesäure, 80 %, in 130 ml Dichlormethan. Man erhält so 11,23 g (82,5 % der Theorie) 2,2,5,5-Tetramethyl-3-pyrrolin-1-oxyl-3-carbonsäure-(5-hydroxy-2,2-dimethyl-1,3-dioxepan-6-yl)-amid, das aus Diethylether/Hexan kristallisiert, einen Schmelzpunkt von 132 bis 133 °C zeigt.

| | | | | | | |
|---|---|---|---|---|---|---|
| $C_{16}H_{27}N_2O_5$ | (327,40) | | | | | |
| Ber.: | 58,70 | C | 8,31 | H | 8,56 | N |
| Gef.: | 58,04 | C | 8,59 | H | 8,34 | N |

Beispiel 22

2,2,5,5-Tetramethyl-3-pyrrolin-1-oxyl-3-carbonsäure-(1,3,4-trihydroxybut-2-yl)-amid

In Analogie zu Beispiel 9 werden 12,53 g (38,27 m mol) 2,2,5,5-Tetramethyl-3-pyrrolin-1-oxyl-3-carbonsäure-(5-hydroxy-2,2-dimethyl-1,3-dioxepan-6-yl)-amid in 100 ml destilliertem Wasser, in dem 0,1 ml (3,6 m mol) konzentrierte Schwefelsäure enthalten sind, verseift. Das Produkt kristallisiert aus der eingeengten wäßrigen Lösung. Man erhält so 9,60 g (87,3 % der Theorie) 2,2,5,5-Tetramethyl-3-pyrrolin-1-oxyl-3-carbonsäure-(1,3,4-trihydroxybut-2-yl)-amid vom Schmelzpunkt 75 bis 77 °C.

| $C_{13}H_{23}N_2O_5$ | (287,34) | | |
|---|---|---|---|
| Ber. : | 54,34 C | 8,07 H | 9,75 N |
| Gef. : | 54,36 C | 8,32 H | 9,60 N |

Beispiel 23

2,2,5,5-Tetramethylpyrrolidin-1-oxyl-3-carbonsäure-(5-hydroxy-2,2-dimethyl-1,3-dioxepan-6-yl)-amid

In Analogie zu Beispiel 4 werden umgesetzt :

24,23 g (130,11 m mol) 2,2,5,5-Tetramethylpyrrolidin-1-oxyl-3-carbonsäure, 13,59 g (133,0 m mol) Triethylamin, 14,87 g (133,0 m mol) Chlorameisensäureethylester, 21,44 g (133 m mol) 6-Amino-2,2-dimethyl-1,3-dioxepan-5-ol in 350 ml absolutem Tetrahydrofuran. Man erhält so 24,83 g (56,7 % der Theorie) 2,2,5,5-Tetramethylpyrrolidin-1-oxyl-3-carbonsäure-(5-hydroxy-2,2-dimethyl-1,3-dioxepan-6-yl)-amid, das aus Diethylether kristallisiert, einen Schmelzpunkt von 190 bis 191 °C zeigt.

| $C_{16}H_{29}N_2O_5$ | (329,42) | | |
|---|---|---|---|
| Ber. : | 58,34 C | 8,87 H | 8,50 N |
| Gef. : | 58,20 C | 8,99 H | 8,39 N |

Beispiel 24

2,2,5,5-Tetramethylpyrrolidin-1-oxyl-3-carbonsäure-(1,3,4-trihydroxybut-2-yl)-amid

In Analogie zu Beispiel 9 werden 6,0 g (18,21 m mol) 2,2,5,5-Tetramethylpyrrolidin-1-oxyl-3-carbonsäure-(5-hydroxy-2,2-dimethyl-1,3-dioxepan-6-yl)-amid in 100 ml destilliertem Wasser, in dem 0,05 ml (1,8 m mol) konzentrierte Schwefelsäure enthalten sind, verseift. Man erhält so 4,83 g (91,7 % der Theorie) 2,2,5,5-Tetramethylpyrrolidin-1-oxyl-3-carbonsäure-(1,3,4-trihydroxybut-2-yl)-amid als orange gefärbten Sirup.

| $C_{13}H_{25}N_2O_5$ | (289,35) | | |
|---|---|---|---|
| Ber. : | 53,96 C | 8,71 H | 9,68 N |
| Gef. : | 53,98 C | 8,91 H | 9,57 N |

Beispiel 25

2,2,5,5-Tetramethyl-3-pyrrolin-1-oxyl-3-carbonsäure-[N-(2,3,4,5,6-pentahydroxyhexyl)-N-methyl]-amid

In Analogie zu Beispiel 4 werden umgesetzt :

12,90 g (70 m mol) 2,2,5,5-Tetramethyl-3-pyrrolin-1-oxyl-3-carbonsäure mit 7,51 g (73,5 m mol) Triethylamin, 8,22 g 73,5 m mol) Chlorameisensäureethylester in 150 ml absolutem Tetrahydrofuran zum Anhydrid, das zu 14,35 g (73,5 m mol) N-Methylglucamin in 80 ml absolutem Pyridin über eine Fritte gesaugt wird. Man läßt über Nacht rühren, engt im Vakuum ein, entfernt Pyridinreste durch Kodestillation mit Ethanol und reinigt durch Chromatografie an 800 g Kieselgel mit Essigester/Ethanol 2 : 1 als Elutionsmittel. Man erhält so 16,11 g (63,7 % der Theorie) 2,2,5,5-Tetramethyl-3-pyrrolin-1-oxyl-3-carbonsäure-[N-(2,3,4,5,6-pentahydroxyhexyl)-N-methyl]-amid als orangen Sirup.

| $C_{16}H_{29}N_2O_7$ | (361,42) | | |
|---|---|---|---|
| Ber. : | 53,17 C | 8,09 H | 7,75 N |
| Gef. : | 52,93 C | 8,22 H | 7,58 N |

Beispiel 26

2,2,5,5-Tetramethyl-3-pyrrolin-1-oxyl-3-carbonsäure-(1,3-dihydroxyprop-2-yl)-amid

In Analogie zu Beispiel 4 werden umgesetzt :

5,50 g (29,86 m mol) 2,2,5,5-Tetramethyl-3-pyrrolin-1-oxyl-3-carbonsäure, 3,27 g (32 m mol) Triethylamin, 3,58 g (32 m mol) Chlorameisensäureethylester und 2,92 g (32 m mol) 2-Amino-1,3-propandiol in 100 ml absolutem Tetrahydrofuran. Man erhält so 5,27 g (68,6 % der Theorie) 2,2,5,5-Tetramethyl-3-pyrrolin-1-oxyl-3-carbonsäure (1,3-dihydroxyprop-2-yl)-amid, das beim Stehen kristallin wird und einen Schmelzpunkt von 151 bis 153 °C zeigt.

| $C_{12}H_{21}N_2O_4$ | (257,31) | | |
|---|---|---|---|
| Ber. : | 56,01 C | 8,23 H | 10,89 N |
| Gef. : | 55,80 C | 8,49 H | 10,71 N |

Beispiel 27

2,2,5,5-Tetramethylpyrrolidin-1-oxyl-3-carbonsäure-N-(1,3-dihydroxyprop-2-yl)-amid

Ein Gemisch aus 1,00 g (5 m mol) 2,2,5,5-Tetramethylpyrrolidin-1-oxyl-3-carbonsäuremethylester und 0,68 g (7,5 m mol) 2-Amino-1,3-propandiol wird 6 Stunden unter Argon auf 120 °C erwärmt. Nach dem Abkühlen wird in destilliertem Wasser aufgenommen, mit Diethylether extrahiert und durch Behandeln mit Ionenaustauscher Amberlite® IR 120 $H^+$-Form überschüssiges Amin gebunden. Der Austauscher wird abfiltriert, mit Wasser gewaschen, und die vereinigten Wasserphasen werden im Vakuum zur Trockene eingeengt. Man erhält so 790 mg (60,8 % der Theorie) 2,2,5,5-Tetramethylpyrrolidin-1-oxyl-3-carbonsäure-(1,3-dihydroxyprop-2-yl)-amid als orange gefärbtes Öl.

| | | | | | | |
|---|---|---|---|---|---|---|
| $C_{12}H_{23}N_2O_4$ | (259,33) | | | | | |
| Ber.: | 55,58 | C | 8,94 | H | 10,80 | N |
| Gef.: | 55,29 | C | 9,22 | H | 10,72 | N |

Beispiel 28

2,2,5,5-Tetramethyl-3-pyrrolin-1-oxyl-3-carbonsäure-(5-hydroxy-2,2-dimethyl-1,3-dioxepan-6-yl)-amid

Zu Einer Lösung von 1,61 g (10 m mol) 6-Amino-2,2-Dimethyl-1,3-dioxepan-5-ol in 100 ml Dichlormethan wird unter Rühren bei 0 °C die Lösung von 2,563 g (10 m mol) gemischtes Anhydrid aus 2,2,5,5-Tetramethyl-3-pyrrolin-1-oxyl-3-carbonsäure und Chlorameisensäureethylester in 20 ml Dichlormethan getropft. Man läßt 60 Minuten nachrühren, wäscht die Lösung mit gesättigter Natriumbikarbonatlösung, trennt die organische Phase ab, trocknet sie über Natriumsulfat und engt sie im Vakuum ein. Der Rückstand wird aus Diethylether/Hexan kristallisiert. Man erhält so 2,27 g (69,3 % der Theorie) 2,2,5,5-Tetramethyl-3-pyrrolin-1-oxyl-3-carbonsäure-(5-hydroxy-2,2-dimethyl-1,3-dioxepan-6-yl)-amid vom Schmelzpunkt 132 bis 133 °C.

Beispiel 29

Bernsteinsäure-{N-[2-hydroxy-2-(2,2-dimethyl-1,3-dioxolan-4-yl)-ethyl]-N'-(2,2,6,6-tetramethyl-1-oxyl-piperidin-4-yl)} diamid.

Zu einer Lösung von 2,71 g (= 10 m mol) Bernsteinsäure-(2,2,6,6-tetramethyl-1-oxido-4-piperidyl) monoamid in 200 ml absolutem Tetrahydrofuran werden unter Rühren, Kühlung und unter Stickstoffspülung 1,01 g (= 10 m mol) Triethylamin gegeben. Man kühlt auf —5 °C ab und tropft innerhalb 20 Minuten 1,09 g (= 10 m mol) Chlorameisensäuremethylester, gelöst in 10 ml absolutem Tetrahydrofuran unter Rühren zu. Es wird 30 Minuten bei —5 °C gerührt. Dann werden 1,61 g (= 10 m mol) 2-Amino-1-(2,2-dimethyl-1,3-dioxolan-4-yl)-ethanol anteilweise zugegeben. Nach 30 Minuten wird die Kühlung entfernt, und es wird 3 Stunden bei Raumtemperatur gerührt. Dann wird mit 200 ml absolutem Ether verdünnt, vom Feststoff abgesaugt, der nach Waschen mit absolutem Ether verworfen wird. Die organische Lösung wird im Vakuum zur Trockne eingeengt, der Rückstand wird in Dichlormethan aufgenommen, mit wenig eiskalten, halbgesättigten Natriumbikarbonatlösung, gewaschen und nach Trocknen über Natriumsulfat die organische Phase erneut im Vakuum zur Trockne eingeengt. Man erhält 2,6 g (= 63 % der Theorie) eines orangen farbenen Sirups.

| | | | | | | |
|---|---|---|---|---|---|---|
| $C_{20}H_{36}N_3O_6$ | (414,520) | | | | | |
| Ber.: | 57,95 | C | 8,75 | H | 10,14 | N |
| Gef.: | 57,86 | C | 9,00 | H | 10,20 | N |

Beispiel 30

Bernsteinsäure-[N-(2,2,6,6-tetramethyl-1-oxylpiperidin-4-yl)-N'-(2,3,4-trihydroxybutyl)]diamid

In 50 ml Wasser, in dem 0,1 ml konzentrierte Schwefelsäure enthalten sind, werden 2,05 g (= 5 m mol) Bernsteinsäure-{-[2-hydroxy-2-(2,2-diethyl-1,3-dioxolan-4-yl)-ethyl]-N'-(2,2,6,6-tetramethyl-1-oxylpiperidin-4-yl)}diamid suspendiert. Unter Rühren wird 3 Stunden auf 50 °C erwärmt. Dann kühlt man auf Raumtemperatur ab und neutralisiert die Lösung mit Ionenaustauscher Amberlite® IRA 410 ($OH^-$-Form). Der Austauscher wird abfiltriert und die Lösung im Vakuum zur Trockne eingeengt. Man erhält 1,67 g (= 90 % der Theorie) eines orange-farbenen Sirups.

| | | | | | | |
|---|---|---|---|---|---|---|
| $C_{17}H_{32}N_3O_6$ | (374,456) | | | | | |
| Ber.: | 54,53 | C | 8,61 | H | 11,22 | N |
| Gef.: | 54,44 | C | 8,80 | H | 11,02 | N |

Beispiel 31

2,2,6,6-Tetramethyl-1,2,5,6-tetrahydropyridin-1-oxyl-4-carbonsäure-[N-(2,3,4,5,6-pentahydroxyhexyl)-N-methyl]-amid

In Analogie zu Beispiel 4 werden umgesetzt:

7,93 g (40 m mol) 2,2,6,6-Tetramethyl-1,2,5,6-tetrahydropyridin-1-oxyl-4-carbonsäure mit 4,3 g (42,5 m mol) Triethylamin, 4,61 g (42,5 m mol) Chlorameisensäureethylester in 100 ml absolutem Tetrahydrofuran zum gemischten Anhydrid, das zu 8,3 g (42,5 m mol) N-Methylglucamin in 50 ml absolutem Pyridin über eine Fritte gesaugt wird. Man läßt über Nacht rühren, engt im Vakuum ein, entfernt Pyridinreste durch Kodestillation mit Ethanol und reinigt durch Chromatografie an 500 g Kieselgel mit Essigester/Ethanol 2 : 1 als Elutionsmittel. Man erhält so 9,19 g (61,2 % der Theorie) 2,2,6,6-Tetramethyl-1,2,5,6-tetrahydropyridin-1-oxyl-4-carbonsäure-[N-(2,3,4,5,6-pentahydroxyhexyl)-N-methyl]-amid als orangen Sirup.

$C_{17}H_{31}N_2O_7$ (375,45)

| | | | |
|---|---|---|---|
| Ber.: | 54,39 C | 8,32 H | 7,46 N |
| Gef.: | 54,12 C | 8,51 H | 7,30 N |

## Beispiel 32

(1-Oxyl-2,2,6,6-tetramethyl-piperidin-4-yl)-essigsäure-[N-(2,3,4,5,6-pentahydroxyhexyl)-N-methyl]-amid

In Analogie zu Beispiel 4 werden umgesetzt:

8,57 g (40 m mol) (1-Oxyl-2,2,6,6-tetramethyl-piperidin-4-yl)-essigsäure mit 4,3 g (42,5 m mol) Triethylamin, 4,61 g (4,25 m mol) Chlorameisensäureethylester in 100 ml absolutem Tetrahydrofuran zum gemischten Anhydrid, das zu 8,3 g (42,5 m mol) N-Methylglucamin in 50 ml absolutem Pyridin über eine Fritte gesaugt wird. Man arbeitet wie in Beispiel 25 beschrieben auf und erhält so 9,36 g (59,8 % der Theorie) (1-Oxyl-2,2,6,6-tetramethylpiperidin-4-yl)-essigsäure-[N-(2,3,4,5,6-pentahydroxyhexyl)-N-methyl-]-amid als orangen Sirup.

$C_{18}H_{35}N_2O_7$ (391,49)

| | | | |
|---|---|---|---|
| Ber.: | 55,23 C | 9,01 H | 7,16 N |
| Gef.: | 55,44 C | 9,18 H | 7,02 N |

## Beispiel 33

Bernsteinsäure-(1-oxyl-2,2,5,5-tetramethylpyrrolidin-3-yl)-monoamid

Zu einer Lösung von 7,86 g (50 m mol) 2,2,5,5-Tetramethylpyrrolidin-1-oxyl-3-amin in 10 ml Pyridin wird unter Kühlung eine Lösung von 6,20 g (62 m mol) Bernsteinsäureanhydrid in 65 ml absolutem Tetrahydrofuran zugetropft. Nach Rühren über Nacht wird das Lösungsmittel im Vakuum abgezogen, der Rückstand in 250 ml Wasser eingerührt und die Lösung mit Diethylether extrahiert. Die wäßrige Lösung wird im Vakuum auf 30 ml eingeengt und gekühlt. Der ausgeschiedene Feststoff wird abgesaugt und im Vakuum getrocknet. Man erhält so 9,62 g (74,8 % der Theorie) Bernsteinsäure-(1-oxyl-2,2,5,5-tetramethylpyrrolidin-3-yl)-monoamid.

$C_{12}H_{21}N_2O_4$ (257,31)

| | | | |
|---|---|---|---|
| Ber.: | 56,02 C | 8,23 H | 10,89 N |
| Gef.: | 56,18 C | 8,22 H | 11,00 N |

## Beispiel 34

Bernsteinsäure-[N-(1-oxyl-2,2,5,5-tetramethylpyrrolidin-3-yl)-N′-methyl-N′-(2,3,4,5,6-pentahydroxyhexyl)]-diamid

In Analogie zu Beispiel 4 werden umgesetzt:

10,29 g (40 m mol) Bernsteinsäure-(1-oxyl-2,2,5,5-tetramethylpyrrolidin-3-yl)-monoamid mit 4,3 g (42,5 m mol) Triethylamin und 4,61 g (42,5 m mol) Chlorameisensäureethylester in 100 ml absolutem Tetrahydrofuran zum Anhydrid, das zu 8,3 g (42,5 m mol) N-Methylglucamin in 50 ml absolutem Pyridin über eine Fritte gesaugt wird. Man arbeitet wie in Beispiel 25 beschrieben auf und erhält so 10,53 g (62,6 % der Theorie) (Bernsteinsäure [N-(1-oxyl-2,2,5,5-tetramethylpyrrolidin-3)-N′-methyl-N′-(2,3,4,5,6-pentahydroxyhexyl)]-diamid als orange gefärbten Sirup.

$C_{19}H_{36}N_2O_8$ (420,51)

| | | | |
|---|---|---|---|
| Ber.: | 54,27 C | 8,63 H | 6,66 N |
| Gef.: | 54,09 C | 8,47 H | 6,49 N |

## Beispiel 35

Lösung des 2,2,5,5-Tetramethyl-3-pyrrolin-1-oxyl-3-carbonsäure-2,3-dihydroxypropylamid

258,317 g (= 1 mol) 2,2,5,5-Tetramethyl-3-pyrrolin-1-oxyl-3-carbonsäure-2,3-dihydroxypropylamid

werden unter Erwärmen in 600 ml Wasser pro injectione gelöst. Man gibt 1,2 g Tromethanin zu, füllt mit Wasser pro injectione auf 1 000 ml auf und füllt die steril filtrierte neutrale Lösung in Flaschen ab.

Mittel für die NMR-Diagnostik.

Beispiel 36

Herstellung mit Nitroxylen beladenen Liposomen

Nach der in Proc. Nat. Acad. Sci. USA 75, 4194 beschriebenen Vorgehensweise wird ein Lipidgemisch aus 50 Teilen Ei-Phosphatidylcholin und 50 Teilen Cholesterol als Trockensubstanz hergestellt. Hiervon werden 500 mg in 30 ml peroxidfreiem Diethylether gelöst und im Ultraschallbad tropfenweise mit 3 ml einer einmolaren Lösung von 2,2,5,5-Tetramethylpyrrolidin-1-oxyl-3-carbonsäure-(2,3-dihydroxypropyl)-amid in Wasser pro injectione versetzt. Nach beendeter Zugabe setzt man die Ultrabeschallung noch 5 Minuten fort und engt dann im Rotavapor ein. Die erhaltene Mikroemulsion wird mit physiologischer Kochsalzlösung verdünnt und bei 0 °C wiederholt durch Zentrifugieren (20 000 g/20 Minuten) von nicht verkapselten Kontrastmittelanteilen befreit. Dann werden die Liposomen im Multivial gefriergetrocknet. Die Applikation erfolgt als Dispension in physiologischer Kochsalzlösung, die bei intravenöser Injektion in einer Dosis von 2,5-5,0 ml/kg eine gute Kontrastdarstellung der Leber und Milz bewirkt.

Beispiel 37

Herstellung einer Lösung eines Nitroxyl-Protein-Konjugates

77,1 mg (= 0,5 mmol) 2,2,5,5-Tetramethylpyrrolidin-1-oxyl-3-carbonsäure werden in 10 ml Dimethylsulfoxid gelöst. Die Lösung wird gekühlt und unter Rühren tropfenweise mit einer Lösung von 57,6 mg (0,5 mMol) N-Hydroxysuccinimid in 2,0 ml Dimethylsulfoxid, darauf tropfenweise mit einer Lösung von 92,9 mg (0,45 mMol) Dicyclohexylcarbodiimid in 2,5 ml Dimethylsulfoxid versetzt. Nach Rühren über Nacht wird filtriert und ein adäquater Teil der Lösung mit dem gewünschten Molverhältnis langsam zu einer Lösung von IgG in 0,1 molarem Natriumphosphat/Natriumchloridpuffer (pH 8.0), IgG-Konzentration: 2,6-3,4 mg/ml zugefügt. Man läßt 2 Stunden bei Raumtemperatur rühren, trennt die Proteinfraktion über eine Bio-Gel P 30-Säule ab und dialysiert sie nacheinander bei 4 °C 24 Stunden gegen 800 ml 0,9 % Natriumchlorid-0,02 % Natriumazid-Lösung und gegen 800 ml eines 0,1 m Natriumcitrat-Puffers (pH 5.0). Durch Gefriertrocknung und Lagerung bei tiefer Temperatur wird ein stabiles Trockenpräparat erhalten, das bei Bedarf in Wasser pro injectione gelöst wird.

## Patentansprüche

1. Diagnostisches Mittel enthaltend eine Verbindung der allgemeinen Formel I

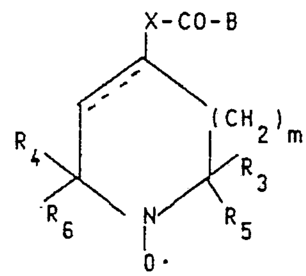

(I)

worin

B einen Protein-, Zucker- oder Lipidrest oder eine Gruppe

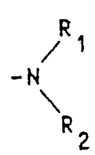

...... eine Einfachbindung oder Doppelbindung bedeutet,

X die Gruppierung $-(CH_2)_n-$ oder falls ...... eine Einfachbindung ist, auch die Gruppierung $-NHCO(CH_2)_n-$ mit n in der Bedeutung von 0 bis 4 darstellt, m die Ziffern 0, 1 oder 2 bedeutet,

$R_1$ einen durch 1 bis 5 Hydroxygruppen, 1 bis 5 $C_2$- bis $C_6$-Acyloxygruppen und/oder 1 bis 3 $C_1$- bis $C_6$-Alkylidendioxygruppen substituierten $C_2$- bis $C_8$-Alkylrest darstellt, $R_2$ die gleiche Bedeutung wie $R_1$ besitzt oder ein Wasserstoffatom oder einen $C_1$- bis $C_4$-Alkylrest bedeutet,

$R_3$ und $R_4$ $C_1$- bis $C_4$-Alkylreste darstellen und

$R_5$ und $R_6$ gegebenenfalls durch 1 bis 3 Hydroxygruppen substituierte $C_1$- bis $C_4$-Alkylreste bedeuten,
zur Verwendung in Diagnostizierverfahren, die am menschlichen oder tierischen Körper vorgenommen werden.

2. Eine Verbindung der allgemeinen Formel I

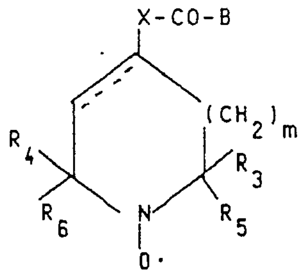

(I)

worin

B einen Protein-, Zucker- oder Lipidrest oder eine Gruppe

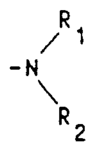

:::::: eine Einfachbindung oder Doppelbindung bedeutet,

X die Gruppierung $-(CH_2)_n-$ oder falls :::::: eine Einfachbindung ist, auch die Gruppierung $-NHCO(CH_2)_n-$ mit n in der Bedeutung von 0 bis 4 darstellt, m die Ziffern 0, 1 oder 2 bedeutet,

$R_1$ einen durch 1 bis 5 Hydroxygruppen, 1 bis 5 $C_2$- bis $C_6$-Acyloxygruppen und/oder 1 bis 3 $C_1$- bis $C_6$-Alkylidendioxygruppen substituierten $C_2$- bis $C_8$-Alkylrest darstellt, $R_2$ die gleiche Bedeutung wie $R_1$ besitzt oder ein Wasserstoffatom oder einen $C_1$- bis $C_4$-Alkylrest bedeutet,

$R_3$ und $R_4$ $C_1$- bis $C_4$-Alkylreste darstellen und

$R_5$ und $R_6$ gegebenenfalls durch 1 bis 3 Hydroxygruppen substituierte $C_1$- bis $C_4$-Alkylreste bedeuten.
zur Verwendung in Diagnostizierverfahren, die am menschlichen oder tierischen Körper vorgenommen werden.

3. Verwendung von Verbindungen der allgemeinen Formel I

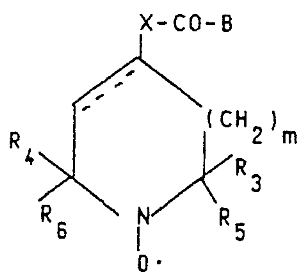

(I)

worin

B einen Protein-, Zucker- oder Lipidrest oder eine Gruppe

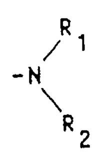

:::::: eine Einfachbindung oder Doppelbindung bedeutet,

X die Gruppierung $-(CH_2)_n-$ oder falls :::::: eine Einfachbindung ist, auch die Gruppierung $-NHCO(CH_2)_n-$ mit n in der Bedeutung von 0 bis 4 darstellt, m die Ziffern 0, 1 oder 2 bedeutet,

$R_1$ einen durch 1 bis 5 Hydroxygruppen, 1 bis 5 $C_2$- bis $C_6$-Acyloxygruppen und/oder 1 bis 3 $C_1$- bis $C_6$-Alkylidendioxygruppen substituierten $C_2$- bis $C_8$-Alkylrest darstellt, $R_2$ die gleiche Bedeutung wie $R_1$ besitzt oder ein Wasserstoffatom oder einen $C_1$- bis $C_4$-Alkylrest bedeutet,

$R_3$ und $R_4$ $C_1$- bis $C_4$-Alkylreste darstellen und

$R_5$ und $R_6$ gegebenenfalls durch 1 bis 3 Hydroxygruppen substituierte $C_1$- bis $C_4$-Alkylreste bedeuten,
zur Herstellung von Mitteln für Diagnostizierverfahren.

4. Verbindungen der allgemeinen Formel I gemäß Patentanspruch 1 mit Ausnahme des 2,2,5,5-Tetramethyl-3-pyrrolin-1-oxyl-3-carbonsäure-(2-hydroxyethyl)-amids und des 2,2,5,5-Tetramethyl-3-pyrrolin-1-oxyl-3-carbonsäure-(2,3-dihydroxypropyl)-amids sowie mit Ausnahme von Verbindungen der allgemeinen Formel I, worin B einen Zucker- oder Phospholipidrest, $R_3$, $R_4$, $R_5$ und $R_6$ einen Alkylrest, m und n die Ziffer 0 sowie ⋯⋯ eine Doppelbindung bedeuten.

5. 2,2,5,5-Tetramethylpyrrolidin-1-oxyl-3-carbonsäure-[2-hydroxy-2-(2,2-dimethyl-1,3-dioxolan-4-yl)-ethyl]-amid.

2,2,5,5-Tetramethylpyrrolidin-1-oxyl-3-carbonsäure-(1,3-dihydroxyprop-2-yl)-amid.

2,2,5,5-Tetramethyl-3-pyrrolin-1-oxyl-carbonsäure-(2,2-dimethyl-1,3-dioxolan-4-yl)-methyl-amid.

2,2,5,5-Tetramethyl-3-pyrrolin-1-oxyl-3-carbonsäure-[N-(2,2-dimethyl-1,3-dioxolan-4-yl-methyl)-N-methyl]-amid.

2,2,5,5-Tetramethyl-3-pyrrolin-1-oxyl-3-carbonsäure-N-(2,3-dihydroxypropyl)-N-methyl-amid.

2,2,5,5-Tetramethylpyrrolidin-1-oxyl-3-carbonsäure-(2,3,4-trihydroxybutyl)-amid.

2,2,5,5-Tetramethylpyrrolidin-1-oxyl-3-carbonsäure-[N-(2,3,4,5,6-pentahydroxyhexyl)-N-methyl]-amid.

2,2,5,5-Tetramethylpyrrolidin-1-oxyl-3-carbonsäure-(2,3-dihydroxypropyl)-amid.

2,2,5,5-Tetramethylpyrrolidin-1-oxyl-3-carbonsäure-bis-(2-hydroxyethyl)-amid.

2,2,5,5-Tetramethylpyrrolidin-1-oxyl-3-carbonsäure-[N-(2,2-dimethyl-1,3-di-oxolan-4-yl-methyl)-N-methyl]-amid.

2,2,5,5-Tetramethylpyrrolidin-1-oxyl-3-carbonsäure-N-(2,3-dihydroxypropyl)-N-methyl-amid.

2,2,5,5-Tetramethyl-3-pyrrolin-1-oxyl-3-carbonsäure-[2-hydroxy-2-(2,2-dimethyl-1,3-dioxolan-4-yl)-ethyl]-amid.

2,2,5,5-Tetramethyl-3-pyrrolin-1-oxyl-3-carbonsäure-(2,3,4-trihydroxybutyl)-amid.

2,2,5,5-Tetramethyl-3-pyrrolin-1-oxyl-3-carbonsäure-(5-hydroxy-2,2-dimethyl-1,3-dioxepan-6-yl)-amid.

2,2,5,5-Tetramethyl-3-pyrrolin-1-oxyl-3-carbonsäure-(1,3,4-trihydroxybut-2-yl)-amid.

2,2,5,5-Tetramethylpyrrolidin-1-oxyl-3-carbonsäure-(5-hydroxy-2,2-dimethyl-1,3-dioxepan-6-yl)-amid.

2,2,5,5-Tetramethylpyrrolidin-1-oxyl-3-carbonsäure-(1,3,4-trihydroxybut-2-yl)-amid.

2,2,5,5-Tetramethyl-3-pyrrolin-1-oxyl-3-carbonsäure-[N-(2,3,4,5,6-pentahydroxyhexyl)-N-methyl]-amid.

2,2,5,5-Tetramethyl-3-pyrrolin-1-oxyl-3-carbonsäure-(1,3-dihydroxyprop-2-yl)-amid.

2,2,5,5-Tetramethyl-3-pyrrolin-1-oxyl-3-carbonsäure-(5-hydroxy-2,2-dimethyl-1,3-dioxepan-6-yl)-amid.

Bernsteinsäure-N-[2-hydroxy-2-(2,2-dimethyl-1,3-dioxolan-4-yl)-ethyl]-N' (2,2,6,6-tetramethyl-1-oxyl-piperidin-4-yl)-diamid.

Bernsteinsäure-[N-(2,2,6,6-tetramethyl-1-oxyl-piperidin-4-yl) N'-(2,3,4-trihydroxybutyl)]-diamid.

(1-Oxyl-2,2,6,6-tetramethyl-piperidin-4-yl)-essigsäure-[N-(2,3,4,5,6-pentahydroxyhexyl)-N-methyl]-amid.

Bernsteinsäure-[N-(1-oxyl-2,2,5,5-tetramethylpyrrolidin-3-yl)-N'-methyl-N'-(2,3,4,5,6-pentahydroxyhexyl)]-diamid.

6. Diagnostisches Mittel, enthaltend eine Verbindung gemäß Anspruch 5 oder 2,2,5,5-Tetramethyl-3-pyrrolin-1-oxyl-3-carbonsäure-(2-hydroxyethyl)-amid oder 2,2,5,5-Tetramethyl-3-pyrrolin-1-oxyl-3-carbonsäure-(2,3-dihydroxypropyl)-amid zur Verwendung in Diagnostizierverfahren, die am menschlichen oder tierischen Körper vorgenommen werden.

7. Diagnostisches Mittel, enthaltend eine Verbindung gemäß Anspruch 5 als Einschlußverbindung in Liposomen.

8. Diagnostisches Mittel, enthaltend eine Verbindung der allgemeinen Formel I, worin ⋯⋯, m, X, $R_1$, $R_2$, $R_3$, $R_4$, $R_5$ und $R_6$ die in Anspruch 1 genannte Bedeutung haben und B einen Zuckerrest darstellt zur Verwendung in Diagnostizierverfahren, die am menschlichen oder tierischen Körper vorgenommen werden.

9. Diagnostisches Mittel, enthaltend eine Verbindung der allgemeinen Formel I, worin ⋯⋯, m, X, $R_1$, $R_2$, $R_3$, $R_4$, $R_5$ und $R_6$ die in Anspruch 1 genannte Bedeutung haben und B einen Proteinrest darstellt.

10. Verfahren zur Herstellung von Verbindungen der allgemeinen Formel I mit B in der Bedeutung einer $—NR_1R_2$-Gruppe gemäß Patentanspruch 4, dadurch gekennzeichnet, daß man in an sich bekannter Weise

a) eine Verbindung der allgemeinen Formel II

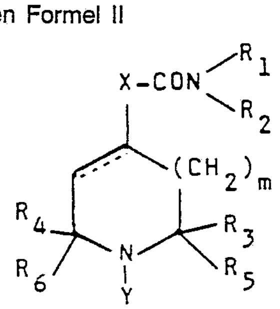

(II)

worin ......, m, X, $R_1$, $R_2$, $R_3$, $R_4$, $R_5$ und $R_6$ die im Anspruch 1 genannte Bedeutung besitzen und Y ein Wasserstoffatom oder eine Hydroxygruppe darstellt, oxidiert, oder

b) eine Carbonsäure der allgemeinen Formel III

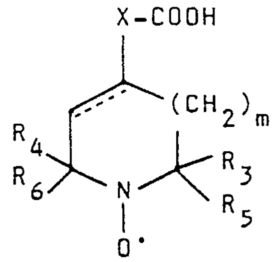

(III)

worin

......, m, X, $R_3$, $R_4$, $R_5$ und $R_6$ die obengenannte Bedeutung besitzen, oder ein reaktionsfähiges Derivat dieser Carbonsäure, mit einem Amin der allgemeinen Formel IV

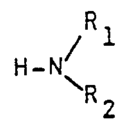

(IV)

worin $R_1$ und $R_2$ die obengenannte Bedeutung besitzen, kondensiert oder

c) eine Carbonsäure der allgemeinen Formel V

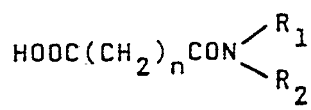

(V)

worin n, $R_1$ und $R_2$ die oben genannte Bedeutung besitzen, oder ein reaktionsfähiges Derivat dieser Carbonsäure, mit einem Amin der allgemeinen Formel VI

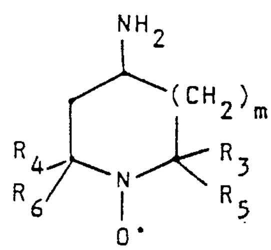

(VI)

worin m, $R_3$, $R_4$, $R_5$ und $R_6$ die obengenannte Bedeutung besitzen, kondensiert und gewünschtenfalls vorhandene Ketalgruppen oder Acylgruppen hydrolytisch spaltet.

11. Verfahren zur Herstellung von Verbindungen der allgemeinen Formel I mit B in der Bedeutung eines Protein-, Zucker- oder Lipidrestes gemäß Anspruch 4, dadurch gekennzeichnet, daß man in an sich bekannter Weise eine Carbonsäure der allgemeinen Formel III, worin ......, m, X, $R_3$, $R_4$, $R_5$ und $R_6$ die im Anspruch 1 genannte Bedeutung besitzen, oder ein reaktionsfähiges Derivat dieser Carbonsäure, mit einer nucleophilen Gruppe eines Proteins, Zuckers oder Lipids umsetzt.

## Claims

1. Diagnostic medium containing a compound of the general formula I

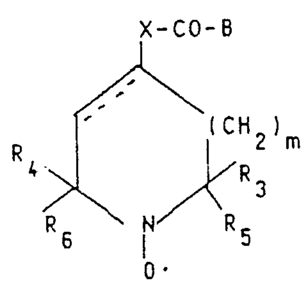

(I)

wherein

B is a protein, sugar or lipid residue or a group

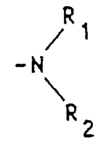

······· is a single bond or a double bond,

X is the grouping $-(CH_2)_n-$ or, if ······ is a single bond, also the grouping $-NHCO(CH_2)_n-$ wherein n represents 0 to 4,

m represents the number 0, 1 or 2,

$R_1$ is a $C_2$- to $C_8$-alkyl radical substituted by from 1 to 5 hydroxy groups, from 1 to 5 $C_2$- to $C_6$-acyloxy groups and/or from 1 to 3 $C_1$- to $C_6$-alkylidenedioxy groups,

$R_2$ has the same meanings as $R_1$ or is a hydrogen atom or a $C_1$- to $C_4$-alkyl radical,

$R_3$ and $R_4$ are $C_1$- to $C_4$-alkyl radicals, and

$R_5$ and $R_6$ are $C_1$- to $C_4$-alkyl radicals optionally substituted by from 1 to 3 hydroxy groups,

for use in diagnostic methods performed on the human or animal body.

2. A compound of the general formula I

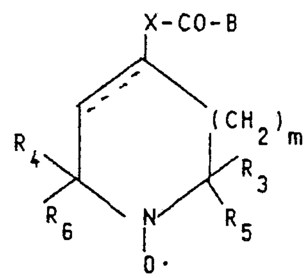

(I)

wherein

B is a protein, sugar or lipid residue or a group

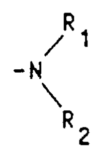

······· is a single bond or a double bond,

X is the grouping $-(CH_2)_n-$ or, if ······ is a single bond, also the grouping $-NHCO(CH_2)_n-$ wherein n represents 0 to 4,

m represents the number 0, 1 or 2,

$R_1$ is a $C_2$- to $C_8$-alkyl radical substituted by from 1 to 5 hydroxy groups, from 1 to 5 $C_2$- to $C_6$-acyloxy groups and/or from 1 to 3 $C_1$- to-$C_6$-alkylidenedioxy groups,

$R_2$ has the same meanings as $R_1$ or is a hydrogen atom or a $C_1$- to $C_4$-alkyl radical,

$R_3$ and $R_4$ are $C_1$- to $C_4$-alkyl radicals, and

$R_5$ and $R_6$ are $C_1$- to $C_4$-alkyl radicals optionally substituted by from 1 to 3 hydroxy groups,

for use in diagnostic methods performed on the human or animal body.

3. The use of compounds of the general formula I

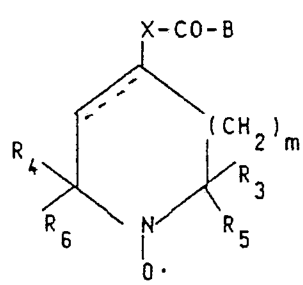

(I)

wherein

B is a protein, sugar or lipid residue or a group

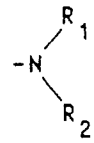

...... is a single bond or a double bond,

X is the grouping $-(CH_2)_n-$ or, if ...... is a single bond, also the grouping $-NHCO(CH_2)_n-$ wherein n represents 0 to 4,

m represents the number 0, 1 or 2,

$R_1$ is a $C_2$- to $C_8$-alkyl radical substituted by from 1 to 5 hydroxy groups, from 1 to 5 $C_2$- to $C_6$-acyloxy groups and/or from 1 to 3 $C_1$- to $C_6$-alkylidenedioxy groups,

$R_2$ has the same meanings as $R_1$ or is a hydrogen atom or a $C_1$- to $C_4$-alkyl radical,

$R_3$ and $R_4$ are $C_1$- to $C_4$-alkyl radicals, and

$R_5$ and $R_6$ are $C_1$- to $C_4$-alkyl radicals optionally substituted by from 1 to 3 hydroxy groups,

for the preparation of media for diagnostic methods.

4. Compounds of the general formula I according to patent claim 1, with the exception of 2,2,5,5-tetramethyl-3-pyrrolin-1-oxyl-3-carboxylic acid (2-hydroxyethyl)-amide and 2,2,5,5-tetramethyl-3-pyrrolin-1-oxyl-3-carboxylic acid (2,3-dihydroxypropyl)-amide and also with the exception of compounds of the general formula I wherein B is a sugar or phospholipid residue, $R_3$, $R_4$, $R_5$ and $R_6$ represent an alkyl radical, m and n represent the number 0 and ...... is a double bond.

5. 2,2,5,5-Tetramethylpyrrolidin-1-oxyl-3-carboxylic acid [2-hydroxy-2-(2,2-dimethyl-1,3-dioxolan-4-yl)-ethyl]-amide.

2,2,5,5-Tetramethylpyrrolidin-1-oxyl-3-carboxylic acid (1,3-dihydroxyprop-2-yl)-amide.

2,2,5,5-Tetramethyl-3-pyrrolin-1-oxyl-carboxylic acid (2,2-dimethyl-1,3-dioxolan-4-yl)-methyl)-amide.

2,2,5,5-Tetramethyl-3-pyrrolin-1-oxyl-3-carboxylic acid [N-(2,2-dimethyl-1,3-dioxolan-4-yl-methyl)-N-methyl]-amide.

2,2,5,5-Tetramethyl-3-pyrrolin-1-oxyl-3-carboxylic acid N-(2,3-dihydroxypropyl)-N-methyl-amide.

2,2,5,5-Tetramethylpyrrolidin-1-oxyl-3-carboxylic acid (2,3,4-trihydroxybutyl)-amide.

2,2,5,5-Tetramethylpyrrolidin-1-oxyl-3-carboxylic acid [N-(2,3,4,5,6-pentahydroxyhexyl)-N-methyl]-amide.

2,2,5,5-Tetramethylpyrrolidin-1-oxyl-3-carboxylic acid (2,3-dihydroxypropyl)-amide.

2,2,5,5-Tetramethylpyrrolidin-1-oxyl-3-carboxylic acid bis-(2-hydroxyethyl)-amide.

2,2,5,5-Tetramethylpyrrolidin-1-oxyl-3-carboxylic acid [N-(2,2-dimethyl-1,3-dioxolan-4-yl-methyl)-N-methyl]-amide.

2,2,5,5-Tetramethylpyrrolidin-1-oxyl-3-carboxylic acid N-(2,3-dihydroxypropyl)-N-methyl-amide.

2,2,5,5-Tetramethyl-3-pyrrolin-1-oxyl-3-carboxylic acid [2-hydroxy-2-(2,2-dimethyl-1,3-dioxolan-4-yl)-ethyl]-amide.

2,2,5,5-Tetramethyl-3-pyrrolin-1-oxyl-3-carboxylic acid (2,3,4-trihydroxybutyl)-amide.

2,2,5,5-Tetramethyl-3-pyrrolin-1-oxyl-3-carboxylic acid (5-hydroxy-2,2-dimethyl-1,3-dioxepan-6-yl)-amide.

2,2,5,5-Tetramethyl-3-pyrrolin-1-oxyl-3-carboxylic acid (1,3,4-trihydroxybut-2-yl)-amide.

2,2,5,5-Tetramethylpyrrolidin-1-oxyl-3-carboxylic acid (5-hydroxy-2,2-dimethyl-1,3-dioxepan-6-yl)-amide.

2,2,5,5-Tetramethylpyrrolidin-1-oxyl-3-carboxylic acid (1,3,4-trihydroxybut-2-yl)-amide.

2,2,5,5-Tetramethyl-3-pyrrolin-1-oxyl-3-carboxylic acid [N-(2,3,4,5,6-pentahydroxyhexyl)-N-methyl]-amide.

2,2,5,5-Tetramethyl-3-pyrrolin-1-oxyl-3-carboxylic acid (1,3-dihydroxyprop-2-yl)-amide.

2,2,5,5-Tetramethyl-3-pyrrolin-1-oxyl-3-carboxylic acid (5-hydroxy-2,2-dimethyl-1,3-dioxepan-6-yl)-amide.

Succinic acid N-[2-hydroxy-2-(2,2-dimethyl-1,3-dioxolan-4-yl)-ethyl]-N'-(2,2,6,6-tetramethyl-1-oxyl-piperidin-4-yl)-diamide.

Succinic acid [N-(2,2,6,6-tetramethyl-1-oxyl-piperidin-4-yl)-N'-(2,3,4-trihydroxybutyl)]-diamide.

(1-Oxyl-2,2,6,6-tetramethyl-piperidin-4-yl)-acetic acid [N-(2,3,4,5,6-pentahydroxyhexyl)-N-methyl]-amide.

Succinic acid [N-(1-oxyl-2,2,5,5-tetramethylpyrrolidin-3-yl)-N'-methyl-N'-(2,3,4,5,6-pentahydroxyhexyl)]-diamide.

6. Diagnostic medium containing a compound according to claim 5 or 2,2,5,5-tetramethyl-3-pyrrolin-1-oxyl-3-carboxylic acid (2-hydroxyethyl)-amide or 2,2,5,5-tetramethyl-3-pyrrolin-1-oxyl-3-carboxylic acid (2,3-dihydroxypropyl)-amide for use in diagnostic methods performed on the human or animal body.

7. Diagnostic medium containing a compound according to claim 5 as an inclusion compound in liposomes.

8. Diagnostic medium containing a compound of the general formula I wherein ......, m, X, $R_1$, $R_2$, $R_3$, $R_4$, $R_5$ and $R_6$ have the meanings indicated in claim 1 and B is a sugar residue, for use in diagnostic methods performed on the human or animal body.

9. Diagnostic medium containing a compound of the general formula I wherein ......, m, X, $R_1$, $R_2$, $R_3$, $R_4$, $R_5$ and $R_6$ have the meanings indicated in claim 1 and B is a protein residue.

10. Process for the preparation of compounds of the general formula I wherein B represents an $-NR_1R_2-$ group according to patent claim 4, characterised in that, in a manner known per se,

a) a compound of the general formula II

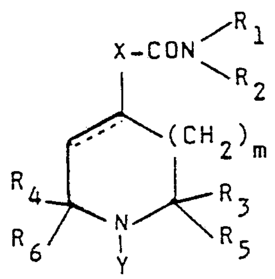

(II)

wherein ......, m, X, $R_1$, $R_2$, $R_3$, $R_4$, $R_5$ and $R_6$ have the meanings indicated in claim 1 and Y is a hydrogen atom or a hydroxy group, is oxidised, or

b) a carboxylic acid of the general formula III

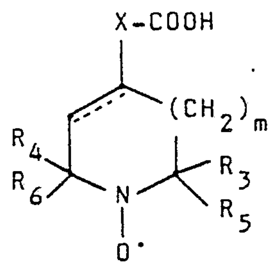

(III)

wherein ......, m, X, $R_3$, $R_4$, $R_5$ and $R_6$ have the meanings indicated above, or a reactive derivative of that carboxylic acid, is condensed with an amine of the general formula IV

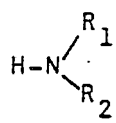

(IV)

wherein $R_1$ and $R_2$ have the meanings indicated above, or

c) a carboxylic acid of the general formula V

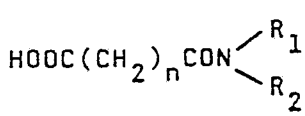

(V)

wherein n, $R_1$ and $R_2$ have the meanings indicated above, or a reactive derivative of that carboxylic acid, is condensed with an amine of the general formula VI

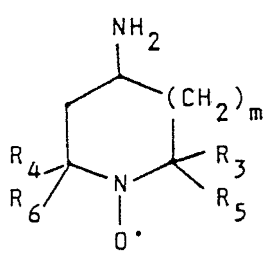

(VI)

wherein m, $R_3$, $R_4$, $R_5$ and $R_6$ have the meanings indicated above, and, if desired, any ketal or acyl groups present are cleaved by hydrolysis.

11. Process for the preparation of compounds of the general formula I wherein B represents a protein, sugar or lipid residue according to claim 4, characterised in that, in a manner known per se, a carboxylic acid of the general formula III wherein ......, m, X, $R_3$, $R_4$, $R_5$ and $R_6$ have the meanings indicated in claim 1, or a reactive derivative of that carboxylic acid, is reacted with a nucleophilic group of a protein, sugar or lipid.

## Revendications

1. Produit pour diagnostic qui contient un composé répondant à la formule générale I :

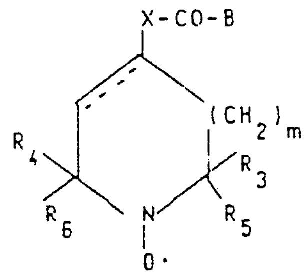

(I)

dans laquelle :

B représente un radical de protéine, de sucre ou de lipide ou un radical :

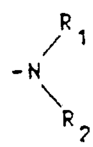

...... représente une liaison simple ou une liaison double,

X représente un radical $-(CH_2)_n-$ ou, dans le cas où le symbole ...... représente une liaison simple, X peut aussi représenter un radical $-NHCO(CH_2)_n-$ dans lequel l'indice n est un nombre de 0 à 4,

m représente un nombre égal à 0, 1 ou à 2,

$R_1$ représente un radical alkyle en $C_2$-$C_8$ qui porte de 1 à 5 radicaux hydroxy, de 1 à 5 radicaux acyloxy en $C_2$-$C_6$ et/ou de 1 à 3 radicaux alkylidène-dioxy en $C_1$-$C_6$,

$R_2$ a la signification qui a été donnée pour $R_1$ ou représente un atome d'hydrogène ou un radical alkyle en $C_1$-$C_4$,

$R_3$ et $R_4$ représentent chacun un radical alkyle en $C_1$-$C_4$ et

$R_5$ et $R_6$ représentent chacun un radical alkyle en $C_1$-$C_4$ éventuellement porteur de 1 à 3 radicaux hydroxy, destiné à être utilisé dans des méthodes de diagnostic appliquées au corps de l'homme ou de l'animal.

2. Composé répondant à la formule générale I :

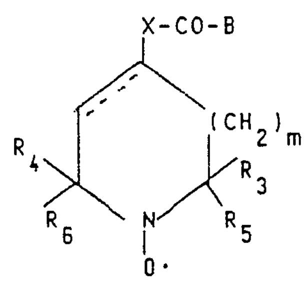

(I)

dans laquelle :

B représente un radical de protéine, de sucre ou de lipide ou un radical :

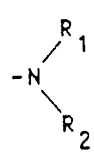

...... représente une liaison simple ou une liaison double,

X représente un radical $—(CH_2)_n—$ ou, dans le cas où le symbole ...... représente une liaison simple, X peut aussi représenter un radical $—NHCO(CH_2)_n—$ dans lequel l'indice n est un nombre de 0 à 4,

m représente un nombre égal à 0, à 1 ou à 2,

$R_1$ représente un radical alkyle en $C_2$-$C_8$ qui porte de 1 à 5 radicaux hydroxy, de 1 à 5 radicaux acyloxy en $C_2$-$C_6$ et/ou de 1 à 3 radicaux alkylidène-dioxy en $C_1$-$C_6$,

$R_2$ a la signification qui a été donnée pour $R_1$ ou représente un atome d'hydrogène ou un radical alkyle en $C_1$-$C_4$,

$R_3$ et $R_4$ représentent chacun un radical alkyle en $C_1$-$C_4$ et

$R_5$ et $R_6$ représentent chacun un radical alkyle en $C_1$-$C_4$ éventuellement porteur de 1 à 3 radicaux hydroxy, destiné à être utilisé dans des méthodes de diagnostic appliquées au corps de l'homme ou de l'animal.

3. Application de composés répondant à la formule générale I :

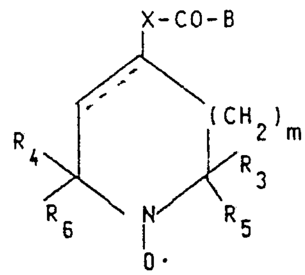

(I)

dans laquelle :

B représente un radical de protéine, de sucre ou de lipide ou un radical :

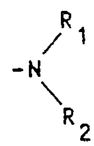

...... représente une liaison simple ou une liaison double,

X représente un radical $—(CH_2)_n—$ ou, dans le cas où le symbole ...... représente une liaison simple, X peut aussi représenter un radical $—NHCO(CH_2)_n—$ dans lequel l'indice n est un nombre de 0 à 4,

m représente un nombre égal à 0, à 1 ou à 2,

$R_1$ représente un radical alkyle en $C_2$-$C_8$ qui porte de 1 à 5 radicaux hydroxy, de 1 à 5 radicaux acyloxy en $C_2$-$C_6$ et/ou de 1 à 3 radicaux alkylidène-dioxy en $C_1$-$C_6$,

$R_2$ a la signification qui a été donnée pour $R_1$ ou représente un atome d'hydrogène ou un radical alkyle en $C_1$-$C_4$,

$R_3$ et $R_4$ représentent chacun un radical alkyle en $C_1$-$C_4$ et

$R_5$ et $R_6$ représentent chacun un radical alkyle en $C_1$-$C_4$ éventuellement porteur de 1 à 3 radicaux hydroxy, à la fabrication de produits pour des méthodes de diagnostic.

4. Composés de formule générale I selon la revendication 1 sauf l'(hydroxy-2 éthyl)-amide de l'acide oxyl-1 tétraméthyl-2,2,5,5 $\Delta^3$-pyrroline-carboxylique-3, le (dihydroxy-2,3 propyl)-amide de l'acide oxyl-1 tétraméthyl-2,2,5,5 $\Delta^3$-pyrroline-carboxylique-3 et les composés de formule générale I dans lesquels B représente un radical de sucre ou de phospholipide, $R_3$, $R_4$, $R_5$ et $R_6$ représentent chacun un radical alkyle, m et n sont égaux chacun à 0, et le symbole ...... représente une double liaison.

5. L'[hydroxy-2(diméthyl-2,2 dioxolanne-1,3 yl-4)-2 éthyl]-amide de l'acide oxyl-1 tétraméthyl-2,2,5,5 pyrrolidine-carboxylique-3 ;

L'(hydroxyméthyl-1 hydroxy-2 éthyl)-amide de l'acide oxyl-1 tétraméthyl-2,2,5,5 pyrrolidine-carboxylique-3.

Le (diméthyl-2,2 dioxolanne-1,3 yl-4 méthyl)-amide de l'acide oxyl-1 tétraméthyl-2,2,5,5 $\Delta^3$-pyrroline-carboxylique-3 ;

Le N-(diméthyl-2,2 dioxolanne-1,3 yl-4 méthyl)-N-méthyl-amide de l'acide oxyl-1 tétraméthyl-2,2,5,5 $\Delta^3$-pyrroline-carboxylique-3 ;

Le N-(dihydroxy-2,3 propyl)-N-méthyl-amide de l'acide oxyl-1 tétraméthyl-2,2,5,5 $\Delta^3$-pyrroline-carboxylique-3 ;

Le (trihydroxy-2,3,4 butyl)-amide de l'acide oxyl-1 tétraméthyl-2,2,5,5 pyrrolidine-carboxylique-3 ;

Le N-(pentahydroxy-2,3,4,5,6 hexyl)-N-méthylamide de l'acide oxyl-1 tétraméthyl-2,2,5,5 pyrrolidine-carboxylique-3 ;

Le (dihydroxy-2,3 propyl)-amide de l'acide oxyl-1 tétraméthyl-2,2,5,5 pyrrolidine-carboxylique-3 ;

Le bis-(hydroxy-2 éthyl)-amide de l'acide oxyl-1 tétraméthyl-2,2,5,5 pyrrolidine-carboxylique-3 ;

Le N-(diméthyl-2,2 dioxolanne-1,3 yl-4 méthyl)-N-méthyl-amide de l'acide oxyl-1 tétraméthyl-2,2,5,5 pyrrolidine-carboxylique-3 ;

Le N-(dihydroxy-2,3 propyl)-N-méthyl-amide de l'acide oxyl-1 tétraméthyl-2,2,5,5 pyrrolidine-carboxylique-3 ;

Le N-[hydroxy-2(diméthyl-2,2 dioxolanne-1,3 yl-4)-2 éthyl]-amide de l'acide oxyl-1 tétraméthyl-2,2,5,5 $\Delta^3$-pyrroline-carboxylique-3 ;

Le N-(trihydroxy-2,3,4 butyl)-amide de l'acide oxyl-1 tétraméthyl-2,2,5,5 $\Delta^3$-pyrroline-carboxylique-3 ;

L'(hydroxy-5 diméthyl-2,2 dioxépanne-1,3 yl-6)-amide de l'acide oxyl-1 tétraméthyl-2,2,5,5 $\Delta^3$-pyrroline-carboxylique-3 ;

L'(hydroxyméthyl-1 dihydroxy-2,3 propyl)-amide de l'acide oxyl-1 tétraméthyl-2,2,5,5 $\Delta^3$-pyrroline-carboxylique-3 ;

L'(hydroxy-5 diméthyl-2,2 dioxépanne-1,3 yl-6)-amide de l'acide oxyl-1 tétraméthyl-2,2,5,5 pyrrolidine-carboxylique-3 ;

L'(hydroxyméthyl-1 dihydroxy-2,3 propyl)-amide de l'acide oxyl-1 tétraméthyl-2,2,5,5 pyrrolidine-carboxylique-3 ;

Le N-(pentahydroxy-2,3,4,5,6 hexyl)-N-méthyl-amide de l'acide oxyl-1 tétraméthyl-2,2,5,5 $\Delta^3$-pyrroline-carboxylique-3 ;

L'(hydroxyméthyl-1 hydroxy-2 éthyl)-amide de l'acide oxyl-1 tétraméthyl-2,2,5,5 $\Delta^3$-pyrroline-carboxylique-3 ;

L'(hydroxy-5 diméthyl-2,2 dioxépanne-1,3 yl-6)-amide de l'acide oxyl-1 tétraméthyl-2,2,5,5 $\Delta^3$-pyrroline-carboxylique-3 ;

Le N-[hydroxy-2(diméthyl-2,2 dioxolanne-1,3 yl-4)-2 éthyl]-N'-(tétraméthyl-2,2,6,6 oxyl-1 pipéridyl-4)-diamide de l'acide succinique ;

Le N-(tétraméthyl-2,2,6,6 oxyl-1 pipéridyl-4)-N'-trihydroxy-2,3,4 butyl)-diamide de l'acide succinique ;

Le N-(pentahydroxy-2,3,4,5,6 hexyl)-N-méthyl-amide de l'acide (oxyl-1 tétraméthyl-2,2,6,6 pipéridyl-4)-acétique ;

Le N-(oxyl-1 tétraméthyl-2,2,5,5 pyrrolidyl-3)-N'-méthyl-N'-(pentahydroxy-2,3,4,5,6 hexyl)-diamide de l'acide succinique.

6. Produit de diagnostic contenant un composé selon la revendication 5 ou de l'(hydroxy-2 éthyl)-amide de l'acide oxyl-1 tétraméthyl-2,2,5,5 $\Delta^3$-pyrroline-carboxylique-3 ou du (dihydroxy-2,3 propyl)-amide de l'acide oxyl-1 tétraméthyl-2,2,5,5 $\Delta^3$-pyrroline-carboxylique-3, pour l'utilisation dans des méthodes de diagnostic exécutées sur le corps de l'homme ou de l'animal.

7. Produit de diagnostic contenant un composé selon la revendication 5 comme composé d'inclusion dans des liposomes.

8. Produit de diagnostic contenant un composé de formule générale I dans lequel ........, m, X, $R_1$, $R_2$, $R_3$, $R_4$, $R_5$ et $R_6$ ont les significations qui leur ont été données dans la revendication 1 et B représente un radical de sucre, pour l'utilisation dans des méthodes de diagnostic exécutées sur le corps de l'homme ou de l'animal.

9. Produit de diagnostic contenant un composé de formule générale I dans lequel ........, m, X, $R_1$, $R_2$, $R_3$, $R_4$, $R_5$ et $R_6$ ont les significations qui leur ont été données à la revendication 1 et B représente un radical de protéine.

10. Procédé de préparation de composés de formule générale I dans lesquels B représente un radical $-NR_1R_2$ selon la revendication 4, procédé caractérisé en ce que, en opérant de manière connue :

a) on oxyde un composé répondant à la formule générale II :

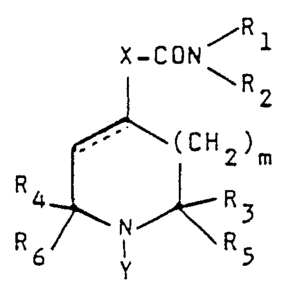

(II)

dans laquelle ........, m, X, $R_1$, $R_2$, $R_3$, $R_4$, $R_5$ et $R_6$ ont les significations qui leur ont été données à la revendication 1, et Y représente un atome d'hydrogène ou un radical hydroxy, ou

b) on condense un acide carboxylique répondant à la formule générale III :

(Voir formule page 26)

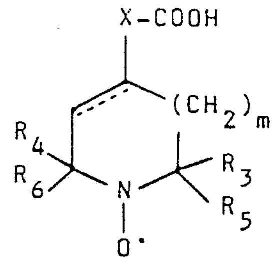

(III)

dans laquelle ......, m, X, $R_3$, $R_4$, $R_5$ et $R_6$ ont les significations qui leur ont été données à la revendication 1, ou un dérivé réactif de cet acide carboxylique, avec une amine répondant à la formule IV :

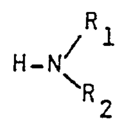

(IV)

dans laquelle $R_1$ et $R_2$ ont les significations qui leur ont été données à la revendication 1, ou

c) on condense un acide carboxylique répondant à la formule générale V :

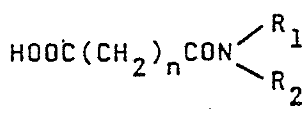

(V)

dans laquelle n, $R_1$ et $R_2$ ont les significations qui leur ont été données à la revendication 1, ou un dérivé réactif de cet acide carboxylique, avec une amine répondant à la formule générale VI :

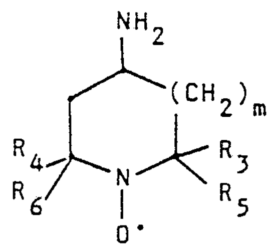

(VI)

dans laquelle m, $R_3$, $R_4$, $R_5$ et $R_6$ ont les significations qui leur ont été données à la revendication 1, et, si on le désire, on coupe par hydrolyse les radicaux acétals ou acyles présents.

11. Procédé de préparation de composés de formule générale I dans lesquels B représente un radical de protéine, de sucre ou de lipide selon la revendication 4, procédé caractérisé en ce qu'on fait réagir, de manière connue, un acide carboxylique de formule générale III dans lequel ...... , m, X, $R_3$, $R_4$, $R_5$ et $R_6$ ont les significations qui leur ont été données à la revendication 1, ou un dérivé réactif d'un tel acide carboxylique, avec un radical nucléophile d'une protéine, d'un sucre ou d'un lipide.